# EUROPEAN PATENT SPECIFICATION

(11) **EP 4 603 497 B1**
(45) Date of publication and mention of the grant of the patent: **02.12.2026**
(21) Application number: 23876817.0
(22) Date of filing: 13.10.2023
(51) Int. Cl.: C07D 401/12, C07K 5/027, A61K 38/06, A61P 31/14, A61K 31/439

(54) **CRYSTAL FORM OF CYANO-SUBSTITUTED POLYPEPTIDE COMPOUND AND PREPARATION METHOD THEREFOR**
KRISTALLFORM EINER CYANOSUBSTITUIERTEN POLYPEPTIDVERBINDUNG UND HERSTELLUNGSVERFAHREN DAFÜR
FORME CRISTALLINE D'UN COMPOSÉ POLYPEPTIDIQUE SUBSTITUÉ PAR CYANO ET SON PROCÉDÉ DE PRÉPARATION

(30) Priority: 14.10.2022 CN 202211263972
(43) Date of publication of application: 20.08.2025
(73) Proprietor: Fujian Akeylink Biotechnology Co., Ltd., Ningde, Fujian 355300 (CN)
(72) Inventor: YANG, Yaxun, Shanghai 200131 (CN); ZHANG, Jianchen, Shanghai 200131 (CN); LI, Peng, Shanghai 200131 (CN); HE, Haiying, Shanghai 200131 (CN); CHEN, Shuhui, Shanghai 200131 (CN)
(74) Representative: Murgitroyd & Company
(86) International application number: PCT/CN2023/124527
(87) International publication number: WO 2024/078618

(56) References cited:
- WO-A1-2022/218442
- WO-A1-2023/043816
- CN-A- 114 466 838
- CN-A- 114 957 383
- ANONYMOUS: "Polymorph screening in pharmaceutical development - European Pharmaceutical Review", 10 August 2010 (2010-08-10), XP093305324, Retrieved from the Internet <URL:https://www.europeanpharmaceuticalreview.com/article/3659/polymorph-screening-in-pharmaceutical-development/>
- S MORISSETTE ET AL: "High-throughput crystallization: polymorphs, salts, co-crystals and solvates of pharmaceutical solids", ADVANCED DRUG DELIVERY REVIEWS, vol. 56, no. 3, 23 February 2004 (2004-02-23), Amsterdam , NL, pages 275 - 300, XP055531004, ISSN: 0169-409X, DOI: 10.1016/j.addr.2003.10.020

## Description

The present application claims priority of the Chinese Patent Application No. CN202211263972.0 filed on October 14, 2022.

### TECHNICAL FIELD

The present disclosure relates to a crystal form of a cyano-substituted polypeptide compound and a preparation method therefor, specifically relates to a crystal form of a compound of formula (I) and a preparation method therefor.

### BACKGROUND

In December 2019, the novel coronavirus disease 2019 (CoVID-19) broke out and quickly evolved into a pandemic that had a significant impact on the global public health system and the global economy. Severe acute respiratory syndrome coronavirus 2 (SARS-CoV-2), as a pathogen, has a very high RNA genome similarity (about 80%) with SARS-CoV-1, which led to the outbreak of SARS in 2003. In response to the prevalence and severity of SARS-CoV-2 infection, many countries and institutions have made great efforts to develop prevention and treatment methods, such as the development of vaccines and large-scale vaccination, as well as the development and application of some therapeutic drugs.

Coronavirus is an enveloped single-stranded positive-sense RNA virus, which can encode structural proteins and non-structural proteins to facilitate the entry of the virus into a host and its replication within the host. In a virus replication cycle, the non-structural protein 3CL (3-chymotrypsin-like protease) protease plays a crucial role, and its main function is to hydrolyze the two polyproteins expressed by the virus. Sequence analysis shows that the 3CL protease may become one of the key targets for drug design.

The 3CL protease currently on the market is Pfizer's Nirmatrelvir tablets, which can reduce hospitalization or mortality by 89% and 70% respectively in high-risk and low-risk groups. It has been approved for marketing or emergency use authorization by the United States and many other countries. At present, there are a number of 3CL proteases at various stages of clinical trials both domestically and internationally. Therefore, the development of 3CL protease inhibitors is particularly important for the treatment of CoVID-19 or other coronavirus infections.

Documents CN 114466838 A & EP 3953330 B1 disclose compounds with antiviral bioactivity.

Document WO 2022/218442 A1 is a P-document from a different applicant, describing compound of formula (I) as antiviral agent.

Document WO 2023/043816 A1 is also P-document from a different applicant, disclosing compound of formula (I) as antiviral agent.

Document CN114957383A describes compounds for use in the treatment of viral infections associated with SARS-CoV-2.

### CONTENT OF THE PRESENT INVENTION

The present disclosure provides a crystal from A of a compound of formula (I), 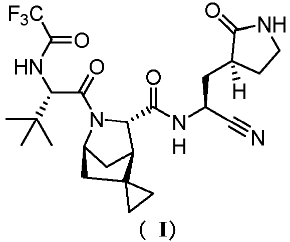 wherein the crystal form A has an X-ray powder diffraction pattern comprising characteristic diffraction peaks at the following 2θ angles: 10.655 ± 0.200°, 11.988 ± 0.200°, 16.055 ± 0.200°, 18.356 ± 0.200°, and 20.083 ± 0.200°.

In some embodiments of the present disclosure, the X-ray powder diffraction pattern of the crystal form A of the compound of formula (I), expressed by a 2θ angle, comprises at least 6, 7, or 8 diffraction peaks selected from: 6.917 ± 0.200°, 10.655 ± 0.200°, 11.988 ± 0.200°, 14.481 ± 0.200°, 16.055 ± 0.200°, 17.653 ± 0.200°, 18.356 ± 0.200°, and 20.083 ± 0.200°.

In some embodiments of the present disclosure, the X-ray powder diffraction pattern of the crystal form A of the compound of formula (I) has characteristic diffraction peaks at the following 2θ angles: 6.917 ± 0.200°, 10.655 ± 0.200°, 11.988 ± 0.200°, 14.481 ± 0.200°, 16.055 ± 0.200°, 17.653 ± 0.200°, 18.356 ± 0.200°, and 20.083 ± 0.200°.

In some embodiments of the present disclosure, the X-ray powder diffraction pattern of the crystal form A of the compound of formula (I), expressed by a 2θ angle, comprises at least 12, 13, 14, 15, or 16 diffraction peaks selected from: 6.917 ± 0.200°, 10.655 ± 0.200°, 11.988 ± 0.200°, 13.837 ± 0.200°, 14.481 ± 0.200°, 16.055 ± 0.200°, 17.653 ± 0.200°, 18.356 ± 0.200°, 20.083 ± 0.200°, 20.839 ± 0.200°, 21.388 ± 0.200°, 22.379 ± 0.200°, 24.577 ± 0.200°, 25.104 ± 0.200°, 26.402 ± 0.200°, and 31.540 ± 0.200°.

In some embodiments of the present disclosure, the X-ray powder diffraction pattern of the crystal form A of the compound of formula (I) has characteristic diffraction peaks at the following 2θ angles: 6.917 ± 0.200°, 10.655 ± 0.200°, 11.988 ± 0.200°, 13.837 ± 0.200°, 14.481 ± 0.200°, 16.055 ± 0.200°, 17.653 ± 0.200°, 18.356 ± 0.200°, 20.083 ± 0.200°, 20.839 ± 0.200°, 21.388 ± 0.200°, 22.379 ± 0.200°, 24.577 ± 0.200°, 25.104 ± 0.200°, 26.402 ± 0.200°, and 31.540 ± 0.200°.

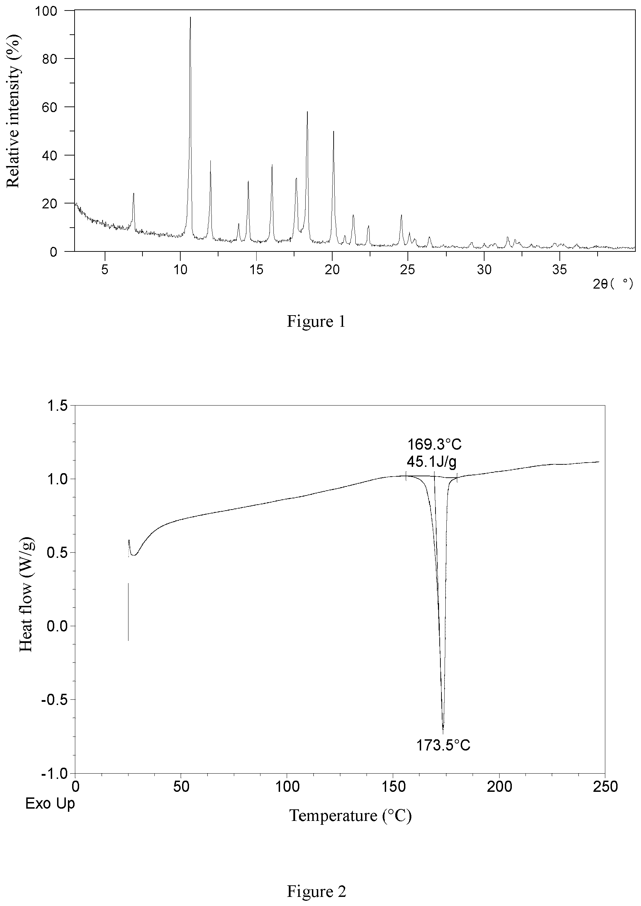

In some embodiments of the present disclosure, the X-ray powder diffraction pattern of the crystal form A of the compound of formula (I) is as shown in Figure 1.

In some embodiments of the present disclosure, the peak position, d-spacing, and relative intensity of the diffraction peaks in the X-ray powder diffraction (XRPD) pattern of the crystal form A of the compound of formula (I) are as shown in Table 1:

**Table 1: Peak position, d-spacing, and relative intensity of diffraction peaks in XRPD pattern of crystal form A**

| **No.** | **2θ angle (°)** | **d-Spacing (Å)** | **Relative intensity (%)** | **No.** | **2θ angle (°)** | **d-Spacing (Å)** | **Relative intensity (%)** |
|---|---|---|---|---|---|---|---|
| 1 | 6.917 | 12.78 | 16.79 | 13 | 24.577 | 3.62 | 13.86 |
| 2 | 10.655 | 8.30 | 100 | 14 | 25.104 | 3.55 | 6.46 |
| 3 | 11.988 | 7.38 | 34.17 | 15 | 25.477 | 3.50 | 3.17 |
| 4 | 13.837 | 6.4 | 8.21 | 16 | 26.402 | 3.38 | 4.68 |
| 5 | 14.481 | 6.12 | 27.65 | 17 | 29.173 | 3.06 | 2.39 |
| 6 | 16.055 | 5.52 | 36.21 | 18 | 30.624 | 2.92 | 1.37 |
| 7 | 17.653 | 5.02 | 29.41 | 19 | 31.540 | 2.84 | 5.08 |
| 8 | 18.356 | 4.83 | 58.85 | 20 | 32.036 | 2.79 | 3.57 |
| 9 | 20.083 | 4.42 | 48.77 | 21 | 34.673 | 2.59 | 2.29 |
| 10 | 20.839 | 4.26 | 3.88 | 22 | 36.116 | 2.49 | 1.71 |
| 11 | 21.388 | 4.15 | 13.79 | 23 | 37.590 | 2.39 | 0.76 |
| 12 | 22.379 | 3.97 | 8.73 | | | | |

In some embodiments of the present disclosure, the crystal form A of the compound of formula (I) has a differential scanning calorimetry (DSC) curve with an endothermic peak at 173.5°C ± 3°C.

In some embodiments of the present disclosure, the crystal form A of the compound of formula (I) has a differential scanning calorimetry curve as shown in Figure 2.

In some embodiments of the present disclosure, the crystal form A of the compound of formula (I) has a thermogravimetric analysis (TGA) curve with a weight loss of 0.00% at 150.0°C ± 3°C.

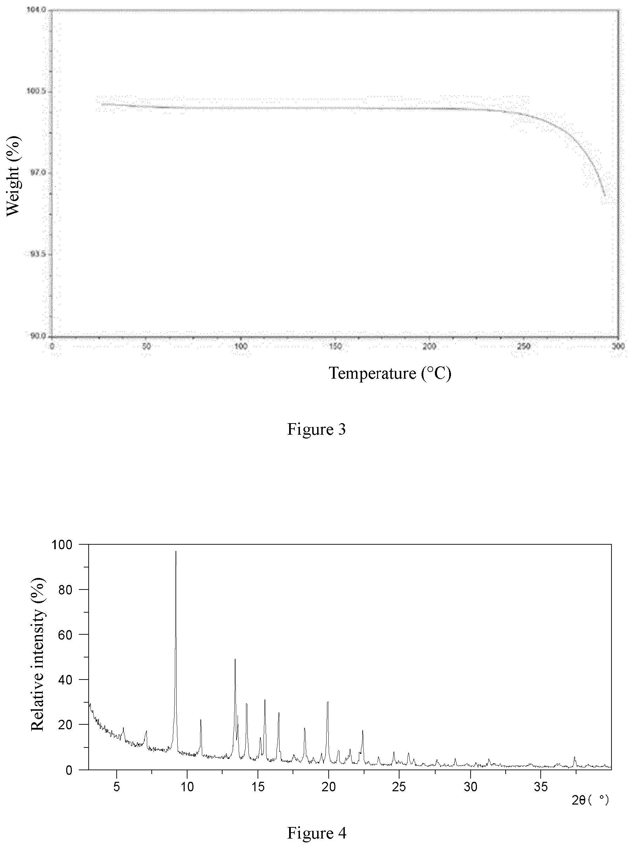

In some embodiments of the present disclosure, the crystal form A of the compound of formula (I) has a thermogravimetric analysis curve as shown in Figure 3.

The present disclosure also provides a method for preparing the crystal form A of the compound of formula (I), which comprises a step of stirring the compound of formula (I) in water. Further, the method also comprises a step of separation.

In some embodiments of the present disclosure, the crystal form A of the compound of formula (I) of the present disclosure is prepared by the following method:
(1) adding water to the compound of formula (I) and stirring;
(2) filtering, and concentrating under reduced pressure to remove water.

In some embodiments of the present disclosure, the stirring in the above step (1) is carried out under heating conditions; preferably, the stirring temperature is 50°C.

The present disclosure also provides a crystal from B of a compound of formula (I), 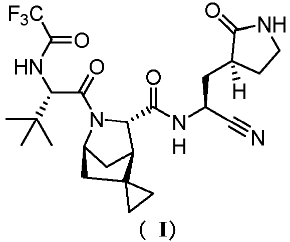 wherein the crystal form B has an X-ray powder diffraction pattern comprising characteristic diffraction peaks at the following 2θ angles: 9.202 ± 0.200°, 13.394 ± 0.200°, 14.212 ± 0.200°, 15.516 ± 0.200°, and 19.917 ± 0.200°.

In some embodiments of the present disclosure, the X-ray powder diffraction pattern of the crystal form B of the compound of formula (I), expressed by a 2θ angle, comprises at least 6, 7, or 8 diffraction peaks selected from: 9.202 ± 0.200°, 10.968 ± 0.200°, 13.394 ± 0.200°, 14.212 ± 0.200°, 15.516 ± 0.200°, 16.483 ± 0.200°, 18.306 ± 0.200°, and 19.917 ± 0.200°.

In some embodiments of the present disclosure, the X-ray powder diffraction pattern of the crystal form B of the compound of formula (I) has characteristic diffraction peaks at the following 2θ angles: 9.202 ± 0.200°, 10.968 ± 0.200°, 13.394 ± 0.200°, 14.212 ± 0.200°, 15.516 ± 0.200°, 16.483 ± 0.200°, 18.306 ± 0.200°, and 19.917 ± 0.200°.

In some embodiments of the present disclosure, the X-ray powder diffraction pattern of the crystal form B of the compound of formula (I), expressed by a 2θ angle, comprises at least 12, 13, 14, 15, or 16 diffraction peaks selected from: 5.459 ± 0.200°, 7.096 ± 0.200°, 9.202 ± 0.200°, 10.968 ± 0.200°, 13.394 ± 0.200°, 14.212 ± 0.200°, 15.516 ± 0.200°, 16.483 ± 0.200°, 18.306 ± 0.200°, 19.488 ± 0.200°, 19.917 ± 0.200°, 20.681 ± 0.200°, 21.526 ± 0.200°, 22.409 ± 0.200°, 24.622 ± 0.200°, and 25.661 ± 0.200°.

In some embodiments of the present disclosure, the X-ray powder diffraction pattern of the crystal form B of the compound of formula (I) has characteristic diffraction peaks at the following 2θ angles: 5.459 ± 0.200°, 7.096 ± 0.200°, 9.202 ± 0.200°, 10.968 ± 0.200°, 13.394 ± 0.200°, 14.212 ± 0.200°, 15.516 ± 0.200°, 16.483 ± 0.200°, 18.306 ± 0.200°, 19.488 ± 0.200°, 19.917 ± 0.200°, 20.681 ± 0.200°, 21.526 ± 0.200°, 22.409 ± 0.200°, 24.622 ± 0.200°, and 25.661 ± 0.200°.

In some embodiments of the present disclosure, the X-ray powder diffraction (XRPD) pattern of the crystal form B of the compound of formula (I) is as shown in Figure 4.

In some embodiments of the present disclosure, the peak position, d-spacing, and relative intensity of the diffraction peaks in the X-ray powder diffraction pattern of the crystal form B of the compound of formula (I) are as shown in Table 2:

**Table 2: Peak position, d-spacing, and relative intensity of diffraction peaks in XRPD pattern of crystal form B**

| **No.** | **2θ angle (°)** | **d-Spacing (Å)** | **Relative intensity (%)** | **No.** | **2θ angle (°)** | **d-Spacing (Å)** | **Relative intensity (%)** |
|---|---|---|---|---|---|---|---|
| 1 | 5.459 | 16.19 | 6.12 | 15 | 20.681 | 4.29 | 6.88 |
| 2 | 7.096 | 12.46 | 8.15 | 16 | 21.526 | 4.13 | 6.21 |
| 3 | 9.202 | 9.61 | 100 | 17 | 22.409 | 3.97 | 16.12 |
| 4 | 10.968 | 8.07 | 18.71 | 18 | 23.536 | 3.78 | 4.5 |
| 5 | 13.394 | 6.61 | 52.93 | 19 | 24.622 | 3.62 | 6.19 |
| 6 | 13.577 | 6.52 | 22.29 | 20 | 25.661 | 3.47 | 6.28 |
| 7 | 14.212 | 6.23 | 29.25 | 21 | 26.047 | 3.42 | 3.06 |
| 8 | 15.196 | 5.83 | 11.18 | 22 | 27.665 | 3.22 | 2.54 |
| 9 | 15.516 | 5.71 | 30.02 | 23 | 28.928 | 3.09 | 4.01 |
| 10 | 16.483 | 5.38 | 24.74 | 24 | 31.320 | 2.86 | 3.46 |
| 11 | 17.563 | 5.05 | 3.55 | 25 | 34.246 | 2.62 | 1.48 |
| 12 | 18.306 | 4.85 | 17.77 | 26 | 36.213 | 2.48 | 1.42 |
| 13 | 19.488 | 4.56 | 5.32 | 27 | 37.360 | 2.41 | 4.84 |
| 14 | 19.917 | 4.46 | 31.59 | 28 | 38.363 | 2.35 | 1.14 |

In some embodiments of the present disclosure, the crystal form B of the compound of formula (I) has a differential scanning calorimetry (DSC) curve with an endothermic peak at 128.4°C ± 3°C.

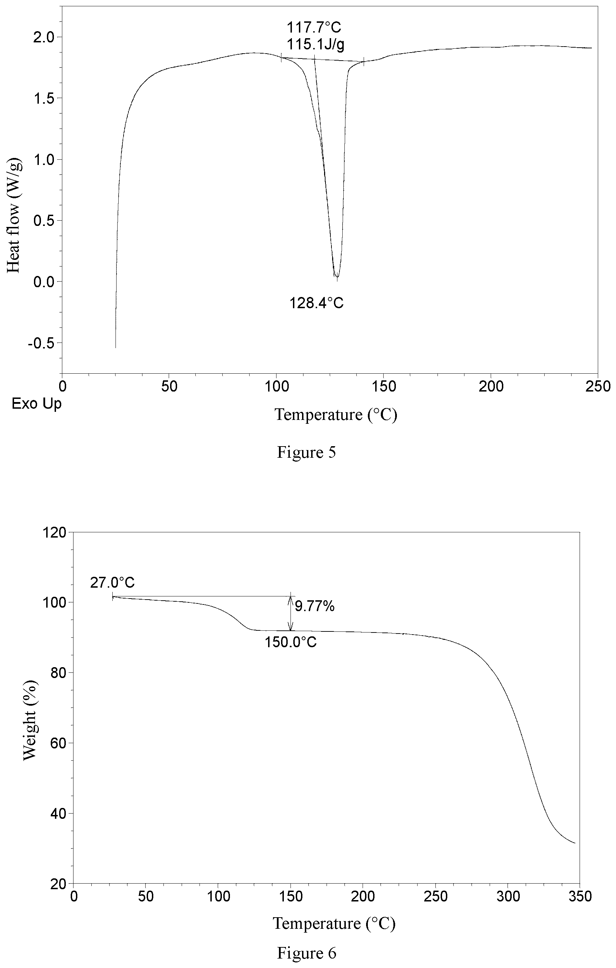

In some embodiments of the present disclosure, the crystal form B of the compound of formula (I) has a differential scanning calorimetry curve as shown in Figure 5.

In some embodiments of the present disclosure, the crystal form B of the compound of formula (I) has a thermogravimetric analysis (TGA) curve with a weight loss of 9.77% at 150.0°C ± 3°C.

In some embodiments of the present disclosure, the crystal form B of the compound of formula (I) has a thermogravimetric analysis curve as shown in Figure 6.

In some embodiments of the present disclosure, the crystal form B of the compound of formula (I) may exist in the form of a solvate crystal.

The present disclosure also provides a method for preparing the crystal form B of the compound of formula (I), which comprises a step of stirring the compound of formula (I) in a mixed solvent of alcohol/n-heptane at room temperature. Further, the method also comprises a step of separation.

In some embodiments of the present disclosure, the crystal form B of the compound of formula (I) of the present disclosure is prepared by the following method:
(1) adding a mixed solvent of alcohol/n-heptane to the compound of formula (I) and stirring at room temperature;
(2) volatilizing at a low temperature and concentrating under reduced pressure.

In some embodiments of the present disclosure, the mixed solvent of alcohol/n-heptane in the above step (1) is preferably ethanol/n-heptane.

In some embodiments of the present disclosure, the ethanol/n-heptane has a preferred solvent ratio (volume ratio) of 1/4.

In some embodiments of the present disclosure, the low temperature in the above step (2) is preferably 5°C.

The present disclosure also provides a crystal from C of a compound of formula (I), 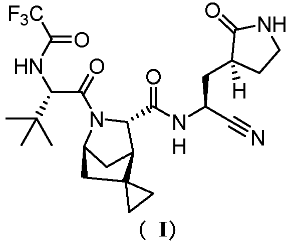 wherein the crystal form C has an X-ray powder diffraction pattern comprising characteristic diffraction peaks at the following 2θ angles: 6.334 ± 0.200°, 8.399 ± 0.200°, 9.435 ± 0.200°, 12.060 ± 0.200°, and 18.126 ± 0.200°.

In some embodiments of the present disclosure, the X-ray powder diffraction pattern of the crystal form C of the compound of formula (I), expressed by a 2θ angle, comprises at least 6, 7, or 8 diffraction peaks selected from: 4.883 ± 0.200°, 6.334 ± 0.200°, 8.399 ± 0.200°, 8.928 ± 0.200°, 9.435 ± 0.200°, 11.168 ± 0.200°, 12.060 ± 0.200°, and 18.126 ± 0.200°.

In some embodiments of the present disclosure, the X-ray powder diffraction pattern of the crystal form C of the compound of formula (I) has characteristic diffraction peaks at the following 2θ angles: 4.883 ± 0.200°, 6.334 ± 0.200°, 8.399 ± 0.200°, 8.928 ± 0.200°, 9.435 ± 0.200°, 11.168 ± 0.200°, 12.060 ± 0.200°, and 18.126 ± 0.200°.

In some embodiments of the present disclosure, the X-ray powder diffraction pattern of the crystal form C of the compound of formula (I), expressed by a 2θ angle, comprises at least 12, 13, 14, 15, or 16 diffraction peaks selected from: 4.883 ± 0.200°, 6.334 ± 0.200°, 8.399 ± 0.200°, 8.928 ± 0.200°, 9.435 ± 0.200°, 11.168 ± 0.200°, 12.060 ± 0.200°, 12.988 ± 0.200°, 14.821 ± 0.200°, 16.182 ± 0.200°, 18.126 ± 0.200°, 18.545 ± 0.200°, 19.474 ± 0.200°, 20.199 ± 0.200°, 24.294 ± 0.200°, and 25.697 ± 0.200°.

In some embodiments of the present disclosure, the X-ray powder diffraction pattern of the crystal form C of the compound of formula (I) has characteristic diffraction peaks at the following 2θ angles: 4.883 ± 0.200°, 6.334 ± 0.200°, 8.399 ± 0.200°, 8.928 ± 0.200°, 9.435 ± 0.200°, 11.168 ± 0.200°, 12.060 ± 0.200°, 12.988 ± 0.200°, 14.821 ± 0.200°, 16.182 ± 0.200°, 18.126 ± 0.200°, 18.545 ± 0.200°, 19.474 ± 0.200°, 20.199 ± 0.200°, 24.294 ± 0.200°, and 25.697 ± 0.200°.

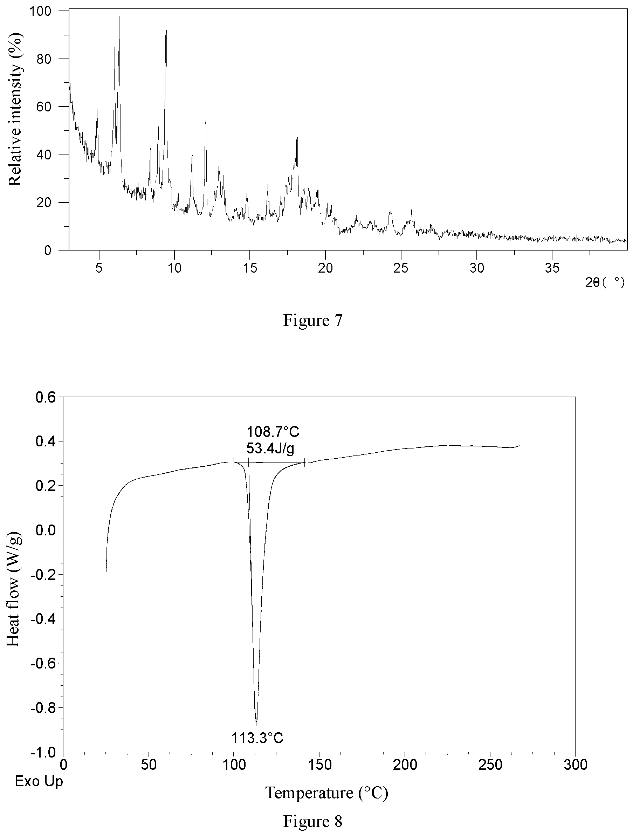

In some embodiments of the present disclosure, for the crystal form C of the compound of formula (I), the X-ray powder diffraction pattern of the crystal form C is as shown in Figure 7.

In some embodiments of the present disclosure, the peak position, d-spacing, and relative intensity of the diffraction peaks in the X-ray powder diffraction pattern of the crystal form C of the compound of formula (I) are as shown in Table 3:

**Table 3: Peak position, d-spacing, and relative intensity of diffraction peaks in XRPD pattern of crystal form C**

| **No.** | **2θ angle (°)** | **d-Spacing (Å)** | **Relative intensity (%)** | **No.** | **2θ angle (°)** | **d-Spacing (Å)** | **Relative intensity (%)** |
|---|---|---|---|---|---|---|---|
| 1 | 4.883 | 18.10 | 29.18 | 11 | 16.182 | 5.48 | 19.85 |
| 2 | 6.040 | 14.63 | 75.51 | 12 | 18.126 | 4.89 | 50.35 |
| 3 | 6.334 | 13.95 | 88.86 | 13 | 18.545 | 4.78 | 22.33 |
| 4 | 8.399 | 10.53 | 31.49 | 14 | 18.902 | 4.70 | 20.71 |
| 5 | 8.928 | 9.91 | 38.51 | 15 | 19.474 | 4.56 | 20.6 |
| 6 | 9.435 | 9.37 | 100 | 16 | 20.199 | 4.40 | 9.50 |
| 7 | 11.168 | 7.92 | 33.89 | 17 | 22.111 | 4.02 | 5.71 |
| 8 | 12.060 | 7.34 | 53.59 | 18 | 24.294 | 3.66 | 12.33 |
| 9 | 12.988 | 6.82 | 26.6 | 19 | 25.697 | 3.47 | 9.56 |
| 10 | 14.821 | 5.98 | 14.53 | | | | |

In some embodiments of the present disclosure, the crystal form C of the compound of formula (I) has a differential scanning calorimetry curve with an endothermic peak at 113.5°C ± 3°C.

In some embodiments of the present disclosure, the crystal form C of the compound of formula (I) has a DSC pattern as shown in Figure 8.

In some embodiments of the present disclosure, the crystal form C of the compound of formula (I) has a thermogravimetric analysis curve with a weight loss of 3.52% at 110.0°C ± 3°C and a weight loss of 10.58% at 150.0°C ± 3°C.

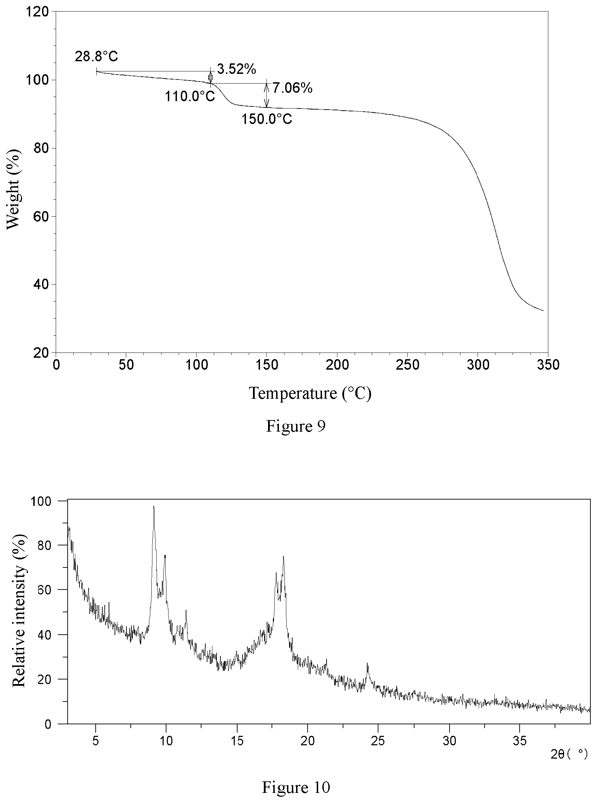

In some embodiments of the present disclosure, the crystal form C of the compound of formula (I) has a TGA pattern as shown in Figure 9.

In some embodiments of the present disclosure, the crystal form C of the compound of formula (I) may exist in the form of a solvate crystal.

The present disclosure also provides a method for preparing the crystal form C of the compound of formula (I), which comprises a step of stirring the compound of formula (I) in a mixed solvent of ethyl acetate/n-heptane at room temperature. Further, the method also comprises a step of separation.

In some embodiments of the present disclosure, the crystal form C of the compound of formula (I) of the present disclosure is prepared by the following method:
(1) adding a mixed solvent of ethyl acetate/n-heptane to the compound of formula (I) and stirring at room temperature;
(2) volatilizing at a low temperature and concentrating under reduced pressure.

In some embodiments of the present disclosure, the ethyl acetate/n-heptane in the above step (1) has a preferred solvent ratio (volume ratio) of 1/4.

In some embodiments of the present disclosure, the low temperature in the above step (2) is preferably -20°C.

The present disclosure also provides a crystal from D of a compound of formula (I), 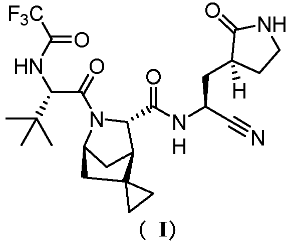 wherein the crystal form D has an X-ray powder diffraction pattern comprising characteristic diffraction peaks at the following 2θ angles: 9.136 ± 0.200°, 9.908 ± 0.200°, 17.787 ± 0.200°, 18.329 ± 0.200°, and 24.298 ± 0.200°.

In some embodiments of the present disclosure, the X-ray powder diffraction pattern of the crystal form D of the compound of formula (I) has characteristic diffraction peaks at the following 2θ angles: 9.136 ± 0.200°, 9.908 ± 0.200°, 17.787 ± 0.200°, 18.329 ± 0.200°, and 24.298 ± 0.200°.

In some embodiments of the present disclosure, the crystal form D of the compound of formula (I) has an XRPD pattern as shown in Figure 10.

In some embodiments of the present disclosure, the peak position, d-spacing, and relative intensity of the diffraction peaks in the X-ray powder diffraction pattern of the crystal form D of the compound of formula (I) are as shown in Table 4:

**Table 4: Peak position, d-spacing, and relative intensity of diffraction peaks in XRPD pattern of crystal form D**

| **No.** | **2θ angle (°)** | **d-Spacing (Å)** | **Relative intensity (%)** |
|---|---|---|---|
| 1 | 9.136 | 9.68 | 100 |
| 2 | 9.908 | 8.93 | 70.31 |
| 3 | 17.787 | 4.99 | 73.90 |
| 4 | 18.329 | 4.84 | 79.93 |
| 5 | 24.298 | 3.66 | 15.70 |

In some embodiments of the present disclosure, the crystal form D of the compound of formula (I) has a differential scanning calorimetry curve with an endothermic peak at 113.3°C ± 3°C.

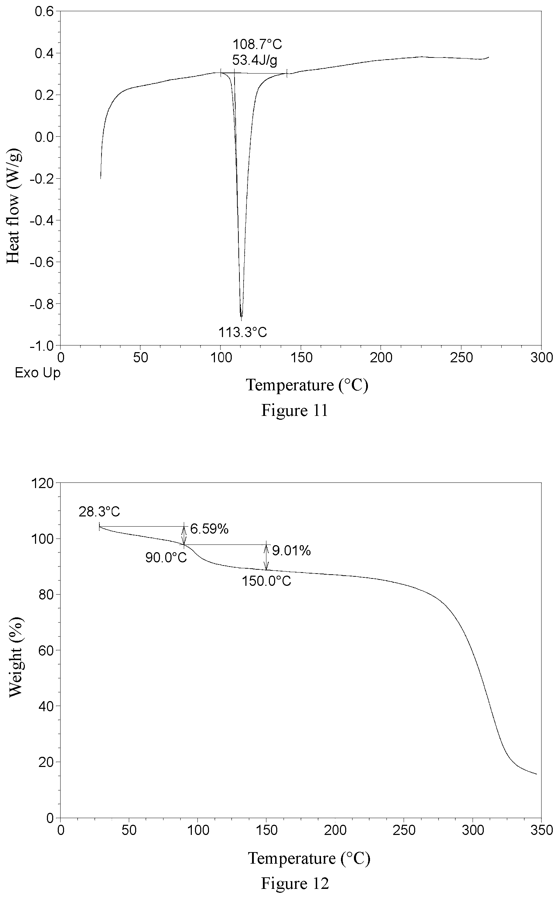

In some embodiments of the present disclosure, the crystal form D of the compound of formula (I) has a DSC pattern as shown in Figure 11.

In some embodiments of the present disclosure, the crystal form D of the compound of formula (I) has a thermogravimetric analysis curve with a weight loss of 6.59% at 90.0°C ± 3°C and a weight loss of 15.60% at 150.0°C ± 3°C.

In some embodiments of the present disclosure, the crystal form D of the compound of formula (I) has a TGA pattern as shown in Figure 12.

In some embodiments of the present disclosure, the crystal form D of the compound of formula (I) may exist in the form of a solvate crystal.

The present disclosure also provides a method for preparing the crystal form D of the compound of formula (I), which comprises a step of stirring the compound of formula (I) in a mixed solvent of dichloromethane/n-heptane at room temperature. Further, the method also comprises a step of separation.

In some embodiments of the present disclosure, the crystal form D of the compound of formula (I) of the present disclosure is prepared by the following method:
(1) adding a mixed solvent of dichloromethane/n-heptane to the compound of formula (I) and stirring at room temperature;
(2) volatilizing at a low temperature and concentrating under reduced pressure.

In some embodiments of the present disclosure, the dichloromethane/n-heptane in the above step (1) has a preferred solvent ratio (volume ratio) of 1/4.

In some embodiments of the present disclosure, the low temperature in the above step (2) is preferably -20°C.

The present disclosure also provides a crystal from E of a compound of formula (I), 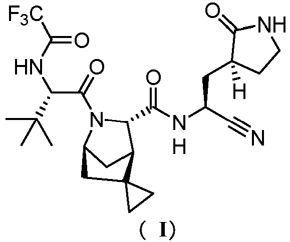 wherein the crystal form E has an X-ray powder diffraction pattern comprising characteristic diffraction peaks at the following 2θ angles: 5.987 ± 0.200°, 9.928 ± 0.200°, 11.998 ± 0.200°, 13.499 ± 0.200°, and 18.044 ± 0.200°.

In some embodiments of the present disclosure, the X-ray powder diffraction pattern of the crystal form E of the compound of formula (I) has characteristic diffraction peaks at the following 2θ angles: 5.987 ± 0.200°, 9.928 ± 0.200°, 11.998 ± 0.200°, 13.499 ± 0.200°, 18.044 ± 0.200°, 19.492 ± 0.200°, and 25.173 ± 0.200°.

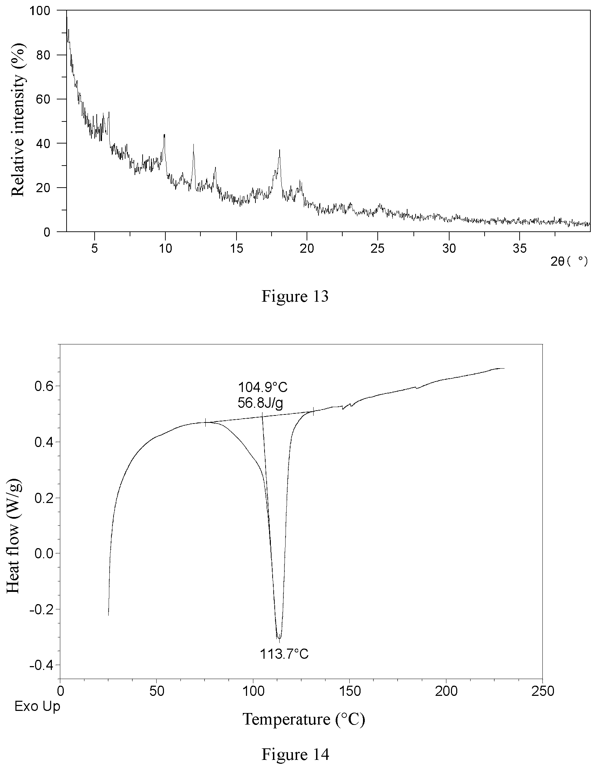

In some embodiments of the present disclosure, the crystal form E of the compound of formula (I) has an XRPD pattern as shown in Figure 13.

In some embodiments of the present disclosure, the peak position, d-spacing, and relative intensity of the diffraction peaks in the X-ray powder diffraction pattern of the crystal form E of the compound of formula (I) are as shown in Table 5:

**Table 5: Peak position, d-spacing, and relative intensity of diffraction peaks in XRPD pattern of crystal form E**

| **No.** | **2θ angle (°)** | **d-Spacing (Å)** | **Relative intensity (%)** |
|---|---|---|---|
| 1 | 5.987 | 14.76 | 59.11 |
| 2 | 9.928 | 8.91 | 85.41 |
| 3 | 11.998 | 7.38 | 81.58 |
| 4 | 13.499 | 6.56 | 51.01 |
| 5 | 18.044 | 4.92 | 100 |
| 6 | 19.492 | 4.55 | 42.19 |
| 7 | 25.173 | 3.54 | 17.41 |

In some embodiments of the present disclosure, the crystal form E of the compound of formula (I) has a differential scanning calorimetry curve with an endothermic peak at 113.7°C ± 3°C.

In some embodiments of the present disclosure, the crystal form E of the compound of formula (I) has a DSC pattern as shown in Figure 14.

In some embodiments of the present disclosure, the crystal form E of the compound of formula (I) has a thermogravimetric analysis curve with a weight loss of 13.97% at 150.0°C ± 3°C.

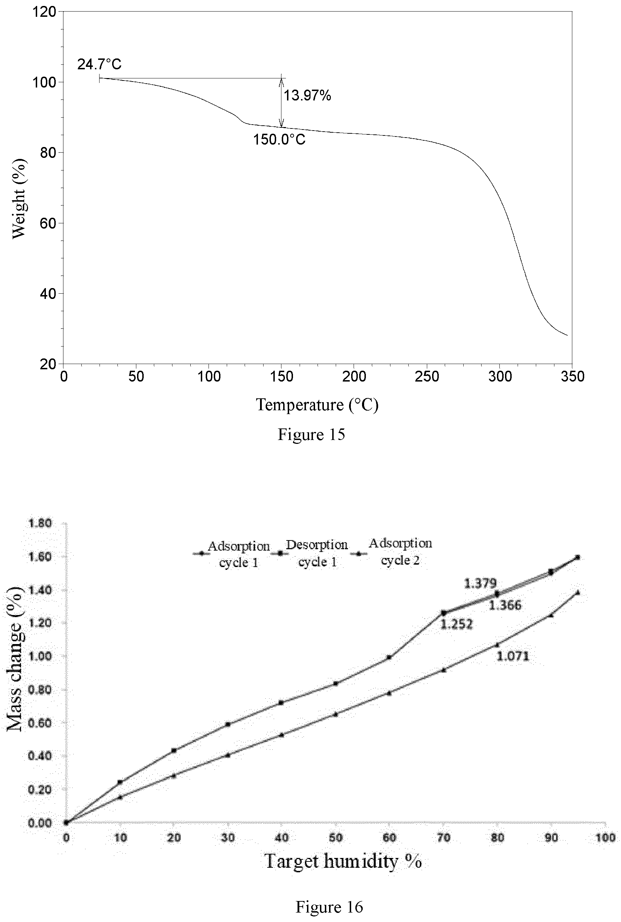

In some embodiments of the present disclosure, the crystal form E of the compound of formula (I) has a TGA pattern as shown in Figure 15.

In some embodiments of the present disclosure, the crystal form E of the compound of formula (I) may exist in the form of a solvate crystal.

The present disclosure also provides a method for preparing the crystal form E of the compound of formula (I), which comprises a step of stirring the compound of formula (I) in toluene at room temperature. Further, the method also comprises a step of separation.

In some embodiments of the present disclosure, the crystal form E of the compound of formula (I) of the present disclosure is prepared by the following method:
(1) adding toluene to the compound of formula (I) and stirring at room temperature;
(2) volatilizing and concentrating under reduced pressure.

In some embodiments of the present disclosure, the volatilizing temperature in the above step (2) is preferably room temperature.

The present disclosure also provides a crystalline composition comprising the crystal form A, the crystal form B, the crystal form C, the crystal form D, or the crystal form E of the compound of formula (I), wherein the crystal form accounts for 50% or more, preferably 80% or more, more preferably 90% or more, and most preferably 95% or more of the crystalline composition by weight.

The present disclosure also provides a pharmaceutical composition, which comprises a therapeutically effective amount of the crystal form A, the crystal form B, the crystal form C, the crystal form D, or the crystal form E of the compound of formula (I), or the above crystalline composition. The pharmaceutical composition of the present disclosure may or may not contain a pharmaceutically acceptable excipient. In addition, the pharmaceutical composition of the present disclosure may further comprise one or more than one other therapeutic agent.

The present disclosure also provides the crystal form A, the crystal form B, the crystal form C, the crystal form D, or the crystal form E of the compound of formula (I), or the above crystalline composition, or the above pharmaceutical composition of the present disclosure for use in treating coronavirus infection.

In some embodiments of the present disclosure, the disease related to coronavirus infection is SARS-CoV-2 virus infection.

### Technical effect

The crystal form of the compound of the present disclosure has good PK properties and therapeutic effects on SARS-CoV-2 virus, possessing a stable crystal form and good hygroscopicity, and is less affected by light and heat.

### Definition and description

Unless otherwise specified, the following terms and phrases used herein have the following meanings. A specific phrase or term should not be considered indefinite or unclear in the absence of a particular definition, but should be understood in the ordinary sense. When a trade name appears herein, it is intended to refer to its corresponding commodity or active ingredient thereof.

For any given crystal form, it is well known in the art of crystallography that the relative intensities of diffraction peaks can change due to preferred orientation caused by factors such as crystal morphology. Where there is an influence of preferred orientation, the peak intensity changes, but the diffraction peak position of the crystal form cannot be changed. Furthermore, there may be a slight error in the position of the peak for any given crystal form, as is also well known in the art of crystallography. For example, due to changes in temperature, movement of the sample, or calibration of the instrument when analyzing the sample, the position of the peak may move, and the 2θ value sometimes has a measurement error of about ± 0.2 degrees. Therefore, it is well known to those skilled in the art that this error should be taken into account when determining each crystal structure.

DSC determines the transition temperature when a crystal absorbs or releases heat due to changes in its crystal structure or melting of the crystal. For the same crystal form of the same compound, the error of thermal transition temperature and melting point is typically within about 5°C or 3°C in continuous analysis. When we say that a compound has a given DSC peak or melting point, it means that the DSC peak or melting point is ± 5°C or ± 3°C. DSC provides an auxiliary method for distinguishing different crystal forms. Different crystal forms can be identified according to their different transition temperature characteristics. It should be noted that for mixtures, the DSC peak or melting point may vary within a wider range. In addition, since the melting process of a substance is accompanied by decomposition, the melting temperature is related to the heating rate.

For the same crystal form, the appearance of TGA weight loss temperature may vary due to factors such as the measuring instrument and the measuring method/conditions. For any specific crystal form, there may be an error in the weight loss temperature, and the error can be about ± 5°C and about ± 3°C.

It should be noted that when preparing drug crystal forms, during the contact process between drug molecules and solvent molecules, it is difficult to avoid the situation that solvent molecules and compound molecules form a eutectic and remain in solid substances due to external conditions and internal factors, thus forming solvates, specifically including stoichiometric solvates and non-stoichiometric solvates. The solvates are included in the scope of the present disclosure.

The "pharmaceutically acceptable excipient" refers to an inert substance that is administered together with the active ingredients and is conducive to the administration of the active ingredients, including but not limited to, any glidants, sweeteners, diluents, preservatives, dyes/colorants, flavor enhancers, surfactants, wetting agents, dispersants, disintegrants, suspending agents, stabilizer agents, isotonic agents, solvents, or emulsifiers which are acceptable for use in humans or animals (such as livestock) and licensed by the China Food and Drug Administration.

The term "crystalline composition" refers to a mixture of the crystal form of the compound of formula (I) of the present disclosure with other crystal forms or amorphous substances of the compound, or other impurities. For example, the crystalline composition of crystal form A of the compound of formula (I) also includes other crystal forms or amorphous substances of the compound of formula (I) or other impurities in addition to crystal form A of the compound of formula (I).

The term "pharmaceutical composition" refers to a mixture of one or more than one compound of the present disclosure or a salt thereof and a pharmaceutically acceptable excipient. The pharmaceutical composition is intended to facilitate the administration of the compound of the present disclosure to an organism.

The therapeutic dose of the compound of the present disclosure may be determined according to, for example, the specific use of treatment, the mode of administration of the compound, the health and condition of the patient, and the judgment of the prescribing physician. The proportion or concentration of the compound of the present disclosure in the pharmaceutical composition may not be fixed, depending on many factors, including dose, chemical properties (such as hydrophobicity), and route of administration.

The term "treatment" means administering the compound or preparation of the present disclosure to improve or eliminate a disease or one or more than one symptom related to the disease, and includes:
(i) inhibiting a disease or disease state, i.e., arresting its progression;
(ii) alleviating a disease or disease state, that is, making the disease or disease state subside.

Unless otherwise claimed in the present disclosure, throughout the specification and subsequent claims, the word "comprise" and its English variants such as "comprises" and "comprising" should to be interpreted in an open-ended, inclusive sense, i.e., "comprising but not limited to".

Throughout this specification, references to "an embodiment" or "embodiments" or "in another embodiment" or "in some embodiments" mean that at least one embodiment includes a specific reference element, structure, or feature related to the embodiment. Therefore, the appearances of the phrases "in an embodiment" or "in embodiments" or "in another embodiment" or "in some embodiments" in different places throughout the specification are not necessarily all referring to the same embodiment. Furthermore, specific elements, structures, or features may be combined in one or more than one embodiment in any suitable manner.

It should be understood that the singular form of the article "a" (corresponding to the English words "a", "an", and "the") used in the specification of the present disclosure and in the appended claims includes plural objects unless the context clearly dictates otherwise. Thus, for example, reference to a reaction including a "catalyst" includes one catalyst, or two or more catalysts. It should also be understood that the term "or" is usually used in its meaning including "and/or" unless the context clearly dictates otherwise.

The chemical reactions of the specific embodiments of the present disclosure are completed in a suitable solvent, and the solvent must be appropriate for the chemical changes of the present disclosure and the required reagents and materials thereof. In order to obtain the compounds of the present disclosure, sometimes it is necessary for those skilled in the art to modify or select synthetic process or reaction schemes on the basis of existing embodiments.

The present disclosure is described in detail by the examples below, but the examples don't constitute any restriction on the present disclosure.

All solvents used in the present disclosure are commercially available and require no further purification before use.

The compounds are named according to the conventional naming principles in the art or by ChemDraw^{®}, and the commercially available compounds use the supplier catalog names.

### Instruments and analytical methods

### 1.1 X-ray powder diffractometer (XRPD) method in the present disclosure

Instrument model: PANalytacal X-ray diffractometer
Test method: About 1 to 2 mg of sample is used for an XRPD test.

The detailed parameters for XRPD are as follows:
Tube: Cu, kα, Kα1 (Å):1.540598, Kα2 (Å):1.544426, Kα2/Kα1 intensity ratio:0.50
Tube voltage: 45 kV, tube current: 40 mA
Divergence slit: 1/8°
Scan mode: Continuous
Scan range (°2Theta): 3 to 40
Scan time per step (s): 46.7
Scan step size (°2Theta): 0.0263
Test time: about 5 minutes

### 1.2 Differential scanning calorimeter (DSC) method in the present disclosure

Instrument model: TA 2500 differential scanning calorimeter
Test method: A sample (about 1 mg) is taken and placed in a DSC aluminum pot for testing. Under the condition of 50 mL/min N₂, the sample is heated from 25°C (room temperature) to a set temperature at a heating rate of 10°C/min.

### 1.3 Thermal gravimetric analyzer (TGA) method in the present disclosure

Instrument model: TA 5500 thermal gravimetric analyzer
Test method: A sample (about 1 mg) is taken and placed in a TGA platinum pot for testing. Under the condition of 25 mL/min N₂, the sample is heated from room temperature to a set temperature at a heating rate of 10°C/min.
Alternatively:
   Thermogravimetric analyzer TGA550
   Test method: A sample (5 to 10 mg) is taken and placed in an aluminum tray built in a TGA platinum pot for testing. Under the condition of 60 mL/min N₂, the sample is heated from room temperature to 300°C at a heating rate of 10°C/min.

### 1.4 Dynamic vapor sorption (DVS) method in the present disclosure

Instrument model: SMS DVS Advantage dynamic vapor sorption analyzer
Test conditions: A sample (10 to 30 mg) is taken and placed in a DVS sample tray for testing.

The detailed parameters for DVS are as follows:
Temperature: 25°C
Equilibrium: dm/dt = 0.002%/min (minimum: 10 minutes, maximum: 180 minutes)
RH (%) range: 70%RH-95%RH-0%RH-95%RH
RH (%) gradient: 10% (90%RH-0%RH-90%RH), 5% (95%RH-90%RH and 90%RH-95%RH).

### BRIEF DESCRIPTION OF THE DRAWINGS

Figure 1 is a Cu-Kα radiation XRPD pattern of a crystal form A of a compound of formula (I);
Figure 2 is a DSC pattern of the crystal form A of the compound of formula (I);
Figure 3 is a TGA pattern of the crystal form A of the compound of formula (I);
Figure 4 is a Cu-Kα radiation XRPD pattern of a crystal form B of the compound of formula (I);
Figure 5 is a DSC pattern of the crystal form B of the compound of formula (I);
Figure 6 is a TGA pattern of the crystal form B of the compound of formula (I);
Figure 7 is a Cu-Kα radiation XRPD pattern of a crystal form C of the compound of formula (I);
Figure 8 is a DSC pattern of the crystal form C of the compound of formula (I);
Figure 9 is a TGA pattern of the crystal form C of the compound of formula (I);
Figure 10 is a Cu-Kα radiation XRPD pattern of a crystal form D of the compound of formula (I);
Figure 11 is a DSC pattern of the crystal form D of the compound of formula (I);
Figure 12 is a TGA pattern of the crystal form D of the compound of formula (I);
Figure 13 is a Cu-Kα radiation XRPD pattern of a crystal form E of the compound of formula (I);
Figure 14 is a DSC pattern of the crystal form E of the compound of formula (I);
Figure 15 is a TGA pattern of the crystal form E of the compound of formula (I);
Figure 16 is a DVS pattern of the crystal form A of the compound of formula (I).

### DETAILED DESCRIPTION OF THE PREFERRED EMBODIMENT

### Example 1: Preparation of compound 1

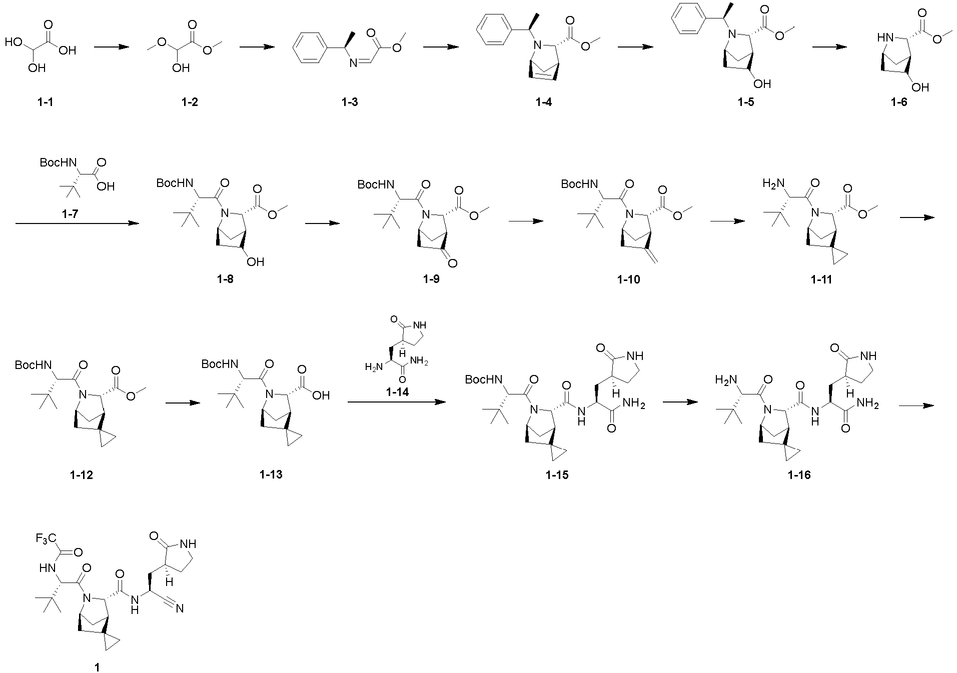

### Step 1: Synthesis of compound 1-2

Compound 1-1 (5 g, 54.32 mmol) was dissolved in methanol (50 mL) and refluxed at 70°C for 48 hours. The reaction system was concentrated under reduced pressure to obtain the crude product of the target product. The crude product was of high purity and was directly used in the next reaction step to obtain compound **1-2.**

¹H NMR (400 MHz, CDCl₃) δ = 4.81 (s, 1H), 3.77 (s, 3H), 3.43 (s, 3H).

### Step 2: Synthesis of compound 1-3

Compound **1-2** was dissolved in toluene (3 mL), cooled to 0°C, then compound (R)-(+)-1-phenethylamine (1.5 g, 12.38 mmol, 1.60 mL) was slowly added dropwise thereto, and the reaction mixture was stirred at 20°C for 1 hour. The reaction system was extracted with ethyl acetate (60 mL) and saturated brine (30 mL) to separate the organic phase, which was dried over anhydrous sodium sulfate and evaporated to dryness by rotary evaporation to obtain the crude product. The crude product was purified by silica gel column chromatography (petroleum ether: ethyl acetate = 1:0 to 5:1) to obtain the target compound **1-3.**

¹H NMR (400 MHz, CDCl₃) δ = 7.95 - 7.56 (m, 1H), 7.31 - 7.17 (m, 5H), 4.71 - 4.40 (m, 1H), 3.95 - 3.71 (m, 3H), 1.67 - 1.51 (m, 3H).

### Step 3: Synthesis of compound 1-4

Compound **1-3** (0.5 g, 2.61 mmol) was dissolved in 2,2,2-trifluoroethanol (5 mL), then trifluoroacetic acid (313.04 mg, 2.75 mmol, 203.28 µL) was added thereto, and the mixture was cooled to -10°C and stirred for 1 hour. The temperature was maintained at -10°C, then cyclopentadiene (207.40 mg, 3.14 mmol) was slowly added dropwise thereto, and the reaction mixture was stirred for another 0.5 hours. The reaction system was concentrated under reduced pressure, added with methyl tert-butyl ether (60 mL) and saturated sodium bicarbonate solution (30 mL × 2), stirred for 10 min, and then extracted to separate the organic phase. The organic phase was dried over anhydrous sodium sulfate and concentrated under reduced pressure. The residue was purified by silica gel column chromatography (petroleum ether: ethyl acetate = 1:0 to 5:1) to obtain compound **1-4,** and the configuration was confirmed by two-dimensional NMR.

¹H NMR (400 MHz, CDCl₃) δ = 7.34 - 7.18 (m, 5H), 6.59 - 6.41 (m, 1H), 6.31 (dd, *J* = 1.6, 5.6 Hz, 1H), 4.35 (br d, *J =* 1.3 Hz, 1H), 3.39 (s, 3H), 3.18 - 3.03 (m, 1H), 2.95 (br s, 1H), 2.33 - 2.22 (m, 1H), 2.14 (br d, *J =* 8.4 Hz, 1H), 1.54 - 1.41 (m, 4H).

MS m/z(ESI): [M+H]⁺ =258.2.

### Step 4: Synthesis of compound 1-5

Compound **1-4** (100.00 mg, 388.61 µmol) was dissolved in tetrahydrofuran (1.25 mL), cooled to -70°C, then borane tetrahydrofuran complex (1 M, 427.47 µL) was slowly added dropwise thereto, and the reaction mixture was slowly warmed to 20°C and stirred for 1 hour. The reaction mixture was cooled to 0°C, added with 10% sodium hydroxide aqueous solution (0.55 mL) and 30% hydrogen peroxide solution (220.28 mg, 1.94 mmol, 186.68 µL), then slowly warmed to 20°C, and stirred for 1 hour. A saturated solution of sodium thiosulfate (10 mL) was added to the reaction system and stirred for 10 min to quench the reaction. Subsequently, saturated brine (20 mL) and ethyl acetate (60 mL × 2) were added thereto for extraction, and the organic phase was separated. A small amount of the sample solution was taken, the pH was adjusted to less than 8 with 3% citric acid, and after the starch potassium iodide paper test was negative, the sample solution was dried over anhydrous sodium sulfate and concentrated under reduced pressure at 30°C. The residue was purified by silica gel column chromatography (petroleum ether: ethyl acetate = 1:0 to 5:1) to obtain compound **1-5.**

¹H NMR (400 MHz, CDCl₃) δ = 7.30 - 7.13 (m, 5H), 3.93 (br d, *J =* 6.5 Hz, 1H), 3.78 (br s, 1H), 3.70 - 3.54 (m, 1H), 3.39 - 3.32 (m, 1H), 3.31 - 3.24 (m, 3H), 2.49 - 2.40 (m, 1H), 2.26 (s, 1H), 2.09 - 2.00 (m, 1H), 1.72 (br d, *J =* 10.1 Hz, 1H), 1.46 (br d, *J =* 6.5 Hz, 1H), 1.41 - 1.33 (m, 3H).

MS m/z(ESI): [M+H]⁺ =276.1.

### Step 5: Synthesis of hydrochloride of compound 1-6

Compound **1-5** (3 g, 10.90 mmol) was dissolved in ethanol (80 mL), and hydrochloric acid (1.19 g, 32.69 mmol) and wet palladium on carbon (15 g, 10.68 mmol) were added thereto. The reaction was stirred at 20°C for 16 hours. The reaction mixture was filtered through diatomite and then directly evaporated to dryness by rotary evaporation to obtain the hydrochloride of crude compound **1-6.**

1H NMR (400 MHz, DMSO-*d*₆) δ = 10.32 - 9.78 (m, 1H), 8.94 - 8.43 (m, 1H), 5.51 - 5.12 (m, 1H), 4.05 - 3.98 (m, 1H), 3.96 - 3.86 (m, 2H), 3.83 - 3.71 (m, 3H), 2.70 - 2.60 (m, 1H), 2.36 - 2.20 (m, 1H), 1.92 - 1.81 (m, 1H), 1.51 - 1.31 (m, 2H)

MS m/z(ESI): [M+H]⁺ = 172.0.

### Step 6: Synthesis of compound 1-8

Compound **1-7** (1.87 g, 10.90 mmol) was dissolved in N,N-dimethylformamide (20 mL), and O-(7-azabenzotriazol-1-yl)-N,N,N,N-tetramethyluronium hexafluorophosphate (4.78 g, 12.58 mmol) and diisopropylethylamine (4.34 g, 33.55 mmol) were added thereto. After stirring for 30 min, the hydrochloride of compound **1-6** (190 mg, 1.12 mmol) was added thereto. The reaction was stirred at 20°C for 16 hours. The reaction mixture was added with water (15 mL) and extracted twice with ethyl acetate (60 mL). The organic phases were combined, washed twice with 5% citric acid (30 mL) and four times with brine (20 mL), dried over anhydrous sodium sulfate, filtered, and evaporated to dryness by rotary evaporation. The residue was purified by column chromatography (petroleum ether: ethyl acetate = 3:1) to obtain compound **1-8.**

¹H NMR (400 MHz, CDCl₃) δ = 5.28 - 5.16 (m, 1H), 4.50 (br s, 1H), 4.28 (d, *J =* 9.8 Hz, 1H), 3.92 (s, 1H), 3.74 (s, 3H), 2.81 (s, 1H), 2.67 (s, 1H), 2.17 (br dd, *J =* 6.1, 12.7 Hz, 1H), 1.99 - 1.93 (m, 1H), 1.90 - 1.84 (m, 1H), 1.59 (br d, *J =* 13.3 Hz, 2H), 1.43 (s, 9H), 1.04 (s, 9H).

MS m/z(ESI): [M+H]⁺ =385.2.

### Step 7: Synthesis of compound 1-9

Compound **1-8** (500 mg, 1.30 mmol) was dissolved in acetonitrile (7.5 mL), and 2-iodoxybenzoic acid (976.31 mg,3.49 mmol) was added thereto, and the reaction mixture was stirred at 60°C for 16 hours. The reaction mixture was directly filtered through diatomite and evaporated to dryness by rotary evaporation. Compound **1-9** was obtained without purification.

MS m/z(ESI): [M-55]⁺ = 327.1.

### Step 8: Synthesis of compound 1-10

Compound **1-9** (0.7 g, 1.83 mmol) was dissolved in tetrahydrofuran (14 mL), and TEBBE (µ-chloro-µ-methylene[bis(cyclopentadienyl)titanium]-dimethylaluminum) reagent (0.5 M, 14.64 mL) was added thereto at 0°C. The reaction mixture was stirred at 0°C for 1 hour, then warmed to 15°C, and stirred for another 3 hours. The reaction mixture was slowly poured into saturated sodium bicarbonate solution (50 mL), filtered through diatomite, extracted with ethyl acetate (30 mL × 3), and washed with saturated brine (30 mL × 2). The residue was purified by column chromatography (petroleum ether: ethyl acetate = 5:1) to obtain compound **1-10.**

1H NMR (400 MHz, DMSO-*d*₆) δ = 6.63 - 6.54 (m, 1H), 5.21 - 5.15 (m, 1H), 4.88 - 4.82 (m, 1H), 4.76 - 4.68 (m, 1H), 4.25 - 4.19 (m, 1H), 3.90 - 3.85 (m, 1H), 3.66 - 3.61 (m, 3H), 3.19 - 3.11 (m, 1H), 2.42 - 2.28 (m, 2H), 1.98 - 1.92 (m, 1H), 1.61 - 1.53 (m, 1H), 1.38 (s, 9H), 1.00 - 0.93 (m, 9H).

MS m/z(ESI): [M+H]⁺ = 381.1.

### Step 9: Synthesis of compound 1-11

Diethylzinc (1 M, 13.14 mL) was slowly added to 1,2-dichloroethane (80 mL) under nitrogen atmosphere at 0°C. The reaction mixture was stirred for 0.25 hours, slowly added with diiodomethane (7.04 g, 26.28 mmol, 2.12 mL) at 0°C, and stirred for 0.25 hours. Trifluoroacetic acid (149.84 mg, 1.31 mmol, 97.30 µL) was slowly added to the reaction system and stirred for another 0.5 hours. Compound **1-10** (0.5 g, 1.31 mmol) in 1,2-dichloroethane (5 mL) was added to the reaction system, warmed to 20°C, and stirred for 12 hours. The reaction was quenched with saturated sodium bicarbonate solution (200 mL), extracted with dichloromethane (100 mL), dried over anhydrous sodium sulfate, and filtered. The filtrate was concentrated. The crude product was purified by preparative HPLC (column type: Phenomenex luna C18 80 * 40 mm * 3 µm; mobile phase: [H₂O(HCl)-acetonitrile]; acetonitrile%: 1% to 30%, 7 min) to obtain compound **1-11.**

MS m/z(ESI): [M+H]⁺ =295.2.

### Step 10: Synthesis of compound 1-12

Compound **1-11** (0.1 g, 339.69 µmol) was dissolved in 1,4-dioxane (3 mL), and then a solution of potassium carbonate (187.79 mg, 1.36 mmol) and di-tert-butyl dicarbonate (111.20 mg, 509.53 µmol, 117.06 µL) in water (1 mL) was added thereto, and the reaction was stirred at 15°C for 12 hours. The reaction mixture was poured into water (30 mL), extracted with ethyl acetate (20 mL × 3), dried over anhydrous sodium sulfate, filtered, and concentrated. The residue was purified by column chromatography (petroleum ether: ethyl acetate = 5:1) to obtain compound **1-12.**

1H NMR (400 MHz, DMSO-*d*₆) δ = 12.52 - 12.16 (m, 1H), 6.56 - 6.31 (m, 1H), 4.66 - 4.58 (m, 1H), 4.24 - 4.18 (m, 1H), 4.02 (s, 1H), 3.36 - 3.28 (m, 1H), 1.97 - 1.91 (m, 2H), 1.81 - 1.73 (m, 2H), 1.66 - 1.59 (m, 1H), 1.36 (s, 9H), 0.99 - 0.93 (m, 9H), 0.80 - 0.70 (m, 1H), 0.64 - 0.53 (m, 1H), 0.49 - 0.33 (m, 2H)

MS m/z(ESI): [M+H]⁺ =395.2.

### Step 11: Synthesis of compound 1-13

Compound **1-12** (88.13 mg, 223.40 µmol) was dissolved in tetrahydrofuran (2 mL) and methanol (0.6 mL), then lithium hydroxide monohydrate (28.12 mg, 670.21 µmol) was dissolved in water (0.6 mL) and added thereto. The reaction was stirred at 15°C for 2 hours. The pH of the reaction mixture was adjusted to 5 with 3% citric acid, extracted with ethyl acetate (20 mL × 3), dried over anhydrous sodium sulfate, filtered, and concentrated to obtain compound **1-13.**

MS m/z(ESI): [M+H]⁺ = 381.3.

### Step 12: Synthesis of compound 1-15

Compound **1-13** (0.056 g, 148.79 µmol) was dissolved in *N,N*-dimethylformamide (2 mL), and *O*-(7-azabenzotriazol-1-yl)-*N,N,N,N*-tetramethyluronium hexafluorophosphate (84.86 mg, 223.18 µmol) was added to the reaction system, and the reaction was stirred at 15°C for 0.5 hours. Then diisopropylethylamine (76.92 mg, 595.16 µmol, 103.67 µL) was added to the reaction mixture, and a solution of hydrochloride of compound **1-14** (43.26 mg, 208.31 µmol) dissolved in N,N-dimethylformamide (0.5 mL) was added to the reaction system, and the reaction was stirred at 15°C for 12 hours. The reaction was diluted with water (20 mL), extracted with ethyl acetate (20 mL × 3), and the organic phase was washed with 3% citric acid (20 mL), washed with saturated sodium chloride solution (20 mL), dried over anhydrous sodium sulfate, filtered, and concentrated to obtain compound **1-15.**

1H NMR (400 MHz, DMSO-*d*₆) δ = 8.22 - 8.08 (m, 1H), 7.56 (s, 1H), 7.32 - 7.16 (m, 1H), 7.01 (s, 1H), 6.51 (br d, J = 9.4 Hz, 1H), 4.60 - 4.49 (m, 1H), 4.28 - 4.17 (m, 2H), 4.14 (s, 1H), 3.18 - 2.98 (m, 2H), 2.47 - 2.35 (m, 1H), 2.17 - 2.09 (m, 2H), 1.96 - 1.84 (m, 2H), 1.76 (br d, J = 11.0 Hz, 1H), 1.71 - 1.42 (m, 4H), 1.39 - 1.34 (m, 9H), 0.95 (s, 8H), 0.84 - 0.77 (m, 1H), 0.73 - 0.65 (m, 1H), 0.39 (br s, 2H)

MS m/z(ESI): [M+H]⁺ = 534.4.

### Step 13: Synthesis of compound 1-16

Compound **1-15** (0.02 g, 37.48 µmol) was dissolved in dichloromethane (2 mL), and trifluoroacetic acid (141.02 mg, 1.24 mmol, 91.57 µL) was added to the reaction system. The reaction mixture was stirred at 15°C for 1 hour. The reaction was directly quenched with sodium bicarbonate solution (10 mL), extracted with dichloromethane (5 mL × 5), and the organic phases were combined and dried over anhydrous sodium sulfate, filtered, and concentrated to obtain compound **1-16.**

1H NMR (400 MHz, DMSO-*d*₆) δ = 8.37 - 8.26 (m, 1H), 8.14 (br d, J = 3.6 Hz, 3H), 7.59 - 7.53 (m, 1H), 7.40 - 7.32 (m, 1H), 7.05 - 6.92 (m, 1H), 4.33 - 4.21 (m, 2H), 3.93 (br d, J = 5.0 Hz, 1H), 3.17 - 3.08 (m, 1H), 3.06 - 2.97 (m, 1H), 2.44 - 2.32 (m, 1H), 2.18 - 2.08 (m, 2H), 1.96 - 1.86 (m, 2H), 1.82 - 1.57 (m, 4H), 1.51 - 1.40 (m, 1H), 1.04 (s, 9H), 0.95 - 0.85 (m, 1H), 0.76 - 0.67 (m, 1H), 0.54 - 0.34 (m, 2H)

MS m/z(ESI): [M+H]⁺ = 434.2.

### Step 14: Synthesis of compound 1

Compound **1-16** (0.03 g, 69.20 µmol) was dissolved in dichloromethane (1 mL), and trifluoroacetic anhydride (58.13 mg, 276.79 µmol, 38.50 µL) was added to the reaction system. The reaction mixture was stirred at 15°C for 1 hour. The reaction was directly quenched with sodium bicarbonate solution (10 mL), extracted with dichloromethane (5 mL × 5), and the organic phases were combined and dried over anhydrous sodium sulfate, filtered, and concentrated. The crude product was separated by preparative HPLC (column type: Waters Xbridge BEH C18 100 * 30 mm * 10 µm; mobile phase: [H₂O(NH₄HCO₃)-acetonitrile]; acetonitrile%: 10% to 50%, 8 min) to obtain compound **1,** that is, compound of formula (I).

1H NMR (400 MHz, DMSO-*d*₆) δ = 9.31 (br d, J = 8.0 Hz, 1H), 8.87 (d, J = 8.6 Hz, 1H), 7.66 (s, 1H), 5.01 - 4.87 (m, 1H), 4.72 - 4.59 (m, 2H), 4.07 (s, 1H), 3.17 - 3.10 (m, 1H), 3.09 - 2.98 (m, 1H), 2.45 - 2.33 (m, 1H), 2.19 - 2.05 (m, 3H), 1.85 - 1.64 (m, 5H), 1.56 (br d, J = 12.0 Hz, 1H), 1.20 - 1.13 (m, 1H), 1.01 (s, 8H), 0.82 - 0.67 (m, 2H), 0.54 - 0.46 (m, 1H), 0.45 - 0.34 (m, 1H)

MS m/z(ESI): [M+H]⁺ = 512.2.

### Example 2: Preparation of crystal form A of compound of formula (I)

The compound of formula (I) (9.4 g, 18.38 mmol) was dissolved in H₂O (235 mL) and stirred at 50°C for 48 hours. The reaction mixture was filtered to obtain a white solid, and the white solid was concentrated under reduced pressure to remove water to obtain crystal form A, which was detected by XRPD.

### Example 3: Preparation of crystal form B of compound of formula (I)

To a glass vial were added the compound of formula (I) (15 mg, 29.32 µmol) and 0.5 mL of ethanol/n-heptane (1:4) solvent. The sample was placed at room temperature with magnetic stirring (1000 rpm) for about 5 days, then cooled to a low temperature (5°C) with stirring to volatilize. The solid was collected by centrifugation and concentrated under reduced pressure to obtain crystal form B, which was detected by XRPD.

### Example 4: Preparation of crystal form C of compound of formula (I)

To a glass vial were added the compound of formula (I) (15 mg, 29.32 µmol) and 0.5 mL of ethyl acetate/n-heptane (1:4) solvent. The sample was placed at room temperature with magnetic stirring (1000 rpm) for about 5 days, then cooled to a low temperature (-20°C) with stirring to volatilize. The solid was collected by centrifugation and concentrated under reduced pressure to obtain crystal form C, which was detected by XRPD.

### Example 5: Preparation of crystal form D of compound of formula (I)

To a glass vial were added the compound of formula (I) (15 mg, 29.32 µmol) and 0.5 mL of dichloromethane/n-heptane (1:4) solvent. The sample was placed at room temperature with magnetic stirring (1000 rpm) for about 5 days, then cooled to a low temperature (-20°C) with stirring to volatilize. The solid was collected by centrifugation and concentrated under reduced pressure to obtain crystal form D, which was detected by XRPD.

### Example 6: Preparation of crystal form E of compound of formula (I)

To a glass vial were added the compound of formula (I) (15 mg, 29.32 µmol) and 0.5 mL of toluene solvent. The sample was placed at room temperature with magnetic stirring (1000 rpm) for about 5 days until it was in a state of dissolved clarification, then cooled to a low temperature (-20°C) and stirred, with no precipitation observed. Then the sample was warmed to room temperature and volatilized to obtain a white solid. The white solid was concentrated under reduced pressure to obtain crystal form E, which was detected by XRPD.

### Example 7: Study on hygroscopicity of crystal form A of compound of formula (I)

### Experimental materials:

### SMS DVS Advantage dynamic vapor sorption analyzer

### Experimental method:

10 to 30 mg of the crystal form A of the compound of formula (I) was put into a DVS sample tray for testing.

### Experimental results:

The DVS pattern of the crystal form A of the compound of formula (I) is shown in Figure 16, with a △W of 0.114%.

### Experimental conclusions:

The crystal form A of the compound of formula (I) exhibits no or almost no hygroscopicity, with a hygroscopic weight gain of 0.114% at 25°C and 80% RH.

### Example 8: Study on stability of crystal form A of compound of formula (I)

The stability test results of crystal form A under different conditions are shown in Table 6.

**Table 6: Stability test under different conditions**

| Condition | Duration (days) | Initial crystal form | Confirmation of crystal form by XRPD after completion of the experiment |
|---|---|---|---|
| Illumination (5000 ± 500 lux (visible light) and 90 µw/cm² (ultraviolet)) | 10 | Crystal form A | Crystal form A |
| High temperature (60°C) | 30 | Crystal form A | Crystal form A |
| High humidity condition (25°C/92.5%RH) | 30 | Crystal form A | Crystal form A |

**Experimental conclusion:** The crystal form A of the compound of the present disclosure has good stability under the conditions of illumination, high temperature, and high humidity.

### Bioassay data:

### Experimental example 1: Evaluation of the in vitro anti-SARS-CoV-2 Mpro protease activity of the test compound

### 1. Experimental materials:

1.1 Reagents and consumables are shown in Table 7.

**Table 7**

| | **Name of reagents and consumables** | **Brand** |
|---|---|---|
| 1 | Tris | Sigma |
| 2 | EDTA | Sigma |
| 3 | NaCl | Sigma |
| 4 | 384 well Plate | Perkin Elmer |
| 5 | Dimethyl sulfoxide (DMSO) | Sigma |
| 6 | Substrate (Dabcyl-KTSAVLQSGFRKM-(Edans)) (SEQ ID NO: 1) | GenScript |
| 7 | SARS-CoV-2 Mpro | WuXi AppTec |
| 8 | GC376 | TargetMol |

1.2 Instruments and brands are shown in Table 8.

**Table 8**

| | Instrument | Brand |
|---|---|---|
| 1 | SpectraMax M2e microplate reader | Molecular Devices |
| 2 | Echo 655 Liquid Handler | Labcyte |
| 3 | Desktop high-speed centrifuge | Eppendorf |

### 2. Experimental method:

The compound was dissolved in DMSO, diluted in a 3-fold gradient using Echo655 according to the required test concentration, with 10 concentration points. Each concentration was duplicated and added to a 384-well plate. The Mpro protein and substrate were diluted with a test buffer (100 mM NaCl, 20 mM Tris-HCl, 1 mM EDTA), and the Mpro protein was added to the 384-well test plate, incubated with the compound at room temperature for 30 min, then the substrate was added. The test concentration of the Mpro protein was 25 nM and the test concentration of the substrate was 25 µM. The mixture was incubated in a constant temperature incubator at 30°C for 60 minutes. Then, the fluorescence signal value at Ex/Em = 340 nm/490 nm was detected using a microplate reader. At the same time, the background well containing substrate and compound but not containing Mpro protein was detected as a control.

### 3. Data analysis:

1) The inhibition rate was calculated using the following formula: 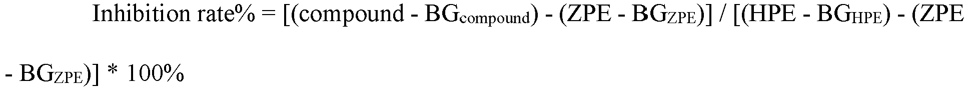 Inhibition rate% = [(compound - BGcompound) - (ZPE - BGZPE)] / [(HPE - BGHPE) - (ZPE - BGZPE)] * 100%
   ^{#} HPE: 100% inhibitory control, containing 25 nM Mpro protein + 25 µM substrate + 1 µM GC376
   ZPE: No inhibitory control, containing 25 nM Mpro protein + 25 µM substrate, containing no compound
   Compound: Test compound well, containing 25 nM Mpro protein + 25 µM substrate + compound
   BG: Background control well, containing 25 µM substrate + compound, and containing no Mpro protein
2) The inhibition rate data (inhibition rate%) of the compound was analyzed using the GraphPad Prism software for log(agonist) vs. response -- Variable slope non-linear fitting, and the IC₅₀ value of the compound was obtained.

**Table 9: In vitro anti-SARS-CoV-2 Mpro protease activity of the test compound**

| **Compound No.** | IC₅₀ (nM) |
|---|---|
| **1** | 1.8 |

Conclusion: The compound of the present disclosure has good *in vitro* anti-SARS-CoV-2 Mpro protease activity.

### Example 2: Evaluation of the in vitro anti-coronavirus activity of the compound using a cytopathic model

1. The experimental materials are shown in Tables 10 and 11.

**Table 10: Reagents and consumables**

| | **Name of reagents and consumables** | **Brand** |
|---|---|---|
| 1 | MEM culture medium | Sigma |
| 2 | L-Glutamine | Gibco |
| 3 | Non-essential amino acids | Gibco |
| 4 | Dual antibody (Penicillin-Streptomycin Solution) | HyClone |
| 5 | Fetal bovine serum (FBS) | ExCell |
| 6 | Phosphate buffered saline (DPBS) | Corning |
| 7 | 0.25% trypsin | Gibco |
| 8 | CellTiter Glo cell activity assay kit | Promega |
| 9 | Remdesivir | MCE |
| 10 | 96-well plate | Grenier |

**Table 11: Instruments**

| | Instrument | Brand |
|---|---|---|
| 1 | Microplate reader | BioTek |
| 2 | Cell counter | Beckman |
| 3 | CO₂ incubator | Thermo |

### 1.1 Cells and viruses

MRC5 cells and coronavirus HCoV OC43 were purchased from ATCC.

MRC5 cells were cultured in MEM (Sigma) culture medium supplemented with 10% fetal bovine serum (Excell), 1% dual antibody (Hyclone), 1% L-glutamine (Gibco), and 1% non-essential amino acids (Gibco). MEM (Sigma) culture medium supplemented with 5% fetal bovine serum (Excell), 1% dual antibody (Hyclone), 1% L-glutamine (Gibco), and 1% non-essential amino acids (Gibco) was used as the experimental culture medium.

### 2. Experimental method

**Table 12: Virus test methods used in this study**

| **Virus (strain)** | **Cell** | **Compound treatment time (days)/endpoint method** | **Control compound** | **Detection reagents** |
|---|---|---|---|---|
| HCoV OC43, 100TCID₅₀/well | 20,000 MRC5 cells/well | 5/CPE | Remdesivir | CellTiter Glo. |

Cells were seeded at a certain density (Table 12) into a 96-well plate and incubated overnight in a 5% CO₂, 37°C incubator. The next day, the diluted compound (8 concentration points, double replicate wells) was added thereto, 50 µL per well. Then the diluted virus was added to each well at 100 TCID₅₀, 50 µL per well. Cell controls (cells, no compound treatment or virus infection), virus controls (cells infected with virus, no compound treatment), and culture medium controls (only culture medium) were set up. The final volume of the experimental culture medium was 200 µL, with a final DMSO concentration of 0.5% in the culture medium. The cells were incubated for 5 days in a 5% CO₂, 33°C incubator. Cell viability was detected using the CellTiter Glo (Promega) cell viability assay kit. The conditions for the cytotoxicity experiment were the same as for the antiviral experiment, but without virus infection.

### 3. Data analysis

The antiviral activity and cytotoxicity of the compound were represented by the inhibition rate (%) and the cell viability (%) of the cytopathic effect caused by the virus at different concentrations of the compound, respectively. The calculation formula is as follows: 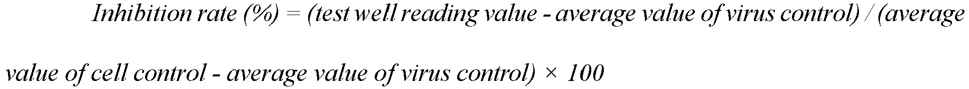 Inhibition rate (%) = (test well reading value - average value of virus control) / (average value of cell control - average value of virus control) × 100 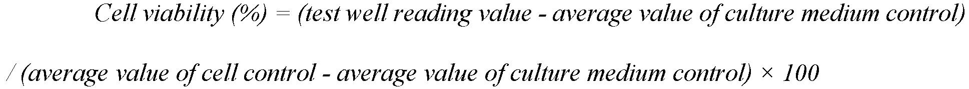 Cell viability (%) = (test well reading value - average value of culture medium control) / (average value of cell control - average value of culture medium control) × 100

The inhibition rate and cell viability of the compound were analyzed using GraphPad Prism for non-linear fitting, and the half-maximal effective concentration (EC₅₀) and half-maximal cytotoxic concentration (CC₅₀) values of the compound were calculated.

**Table 13: In vitro anti-coronavirus activity of the compound using a cytopathic model**

| **Compound No.** | EC₅₀ (nM) | EC₉₀ (nM) | CC₅₀ (nM) |
|---|---|---|---|
| **1** | 5.1 | 7.9 | > 10000 |

Conclusion: The compound of the present disclosure has good *in vitro* anti-coronavirus activity at the cellular level, and has no cytotoxicity.

### Experimental example 3: Pharmacokinetic test of rats

In this study, male and female SD rats were selected as test animals. After a single intravenous injection of 2 mg/kg and a single gavage administration of 30 mg/kg of the crystal form A of compound 1, the plasma drug concentration of the test compound at different time points was quantitatively determined using LC/MS/MS method, so as to evaluate the pharmacokinetic characteristics of the test drug in rats.

Animals were grouped according to 3 per group/gender. After administration of the crystal form A of compound **1,** plasma samples were collected at 0.083 (only for the intravenous injection group), 0.25, 0.5, 1, 2, 4, 6, 8, and 24 hours post-administration. The plasma drug concentration was determined using LC-MS/MS method. The experimental results are shown in Table 14.

**Table 14: Pharmacokinetic parameters in rats**

| **Group** | **1** | | **2** | |
|---|---|---|---|---|
| **Route of administration** | **Intravenous injection** | | **Gavage** | |
| **Vehicle** | **5% DMSO + 40% PEG400 + 55% physiological saline** | | **10% Solutol + 30% PEG400 + 2% Tween 80 + 58% pure water** | |
| **Dose (mg/kg)** | **2** | | **30** | |

| **Pharmacokinetic parameters** | **Male** | **Female** | **Male** | **Female** |
|---|---|---|---|---|
| **C₀ or Cₘₐₓ (ng/mL)** | 2040 | 2060 | 2720 | 2850 |
| **Tₘₐₓ (h)** | -- | -- | 0.917 | 0.500 |
| **Cl (mL/min/kg)** | 42.4 | 42.5 | -- | -- |
| **Vdₛₛ (L/kg)** | 1.11 | 1.04 | -- | -- |
| **AUC₀₋ₗₐₛₜ (ng·h/mL)** | 792 | 778 | 5960 | 9910 |
| **AUC_{0-inf} (ng·h/mL)** | 804 | 785 | 5980 | ND |
| **T_{1/2} (h)** | 0.330 | 0.304 | 0.826 | ND |
| **Bioavailability (%)** | -- | -- | 50.2 | 84.9 |

| | | | | |
|---|---|---|---|---|
| a: Bioavailability was calculated from AUC0-last and theoretical dose. ND: Not determined. --: Not applicable. | | | | |

**Experimental conclusion:** The compound of the present disclosure shows high exposure in plasma and high bioavailability.

### Experimental example 4: Evaluation of the in vivo antiviral efficacy of the test compound using a suckling mouse infection model with the coronavirus OC43 strain

C57BL/6J suckling mice were infected with a lethal dose of coronavirus via intranasal administration, and treated with the vehicle (5% DMSO + 40% PEG400 + 55% water) and compound 1 2 hours before infection. During the experiment, the weight, health status, and survival of the suckling mice were monitored daily to evaluate the protective effect of compound 1 at different doses.

The body weight of the suckling mice in the vehicle group continued to decrease after the 6th day of virus inoculation, with an endpoint survival rate of 0%. Compound 1 (12.5, 25, 50 mpk) was administered for the first time 2 hours before infection, with endpoint survival rates of 87.5%, 100%, and 100%, respectively. The experimental results are shown in Table 15.

**Table 15: Survival analysis of mice**

| **Group** | **Treatment** | | | | **Median survival time (days)** | **Survival rate (%)** | **Log-rank (Mantel-cox) test** |
|---|---|---|---|---|---|---|---|
| | **Compound** | **Administration dose (mg/kg)** | **Regimen** | **Time of first administration** | | | ***p*-value*** |
| **1** | Vehicle | 0 | Once a day for 7 consecutive days | 2 hours before inoculation | 7.5 | 0 | NA |
| **2** | Compound **1** | 12.5 | | | > 14 | 87.5 | < 0.001 |
| **3** | Compound **1** | 25 | | | > 14 | 100 | < 0.001 |
| **4** | Compound **1** | 50 | | | > 14 | 100 | < 0.001 |

| | | | | | | | |
|---|---|---|---|---|---|---|---|
| * Compared with the solvent group | | | | | | | |

**Experimental conclusion:** Compound **1** has good *in vivo* anti-coronaviral efficacy, showing a good dose-effect relationship.

## Claims

1. A crystal form A, B, C, D or E of a compound of formula (I), 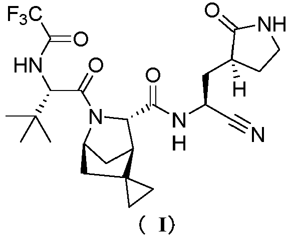
wherein (1) the crystal form A has an X-ray powder diffraction pattern comprising characteristic diffraction peaks at the following 2θ angles: 10.655 ± 0.200°, 11.988 ± 0.200°, 16.055 ± 0.200°, 18.356 ± 0.200°, and 20.083 ± 0.200°;
(2) the crystal form B has an X-ray powder diffraction pattern comprising characteristic diffraction peaks at the following 2θ angles: 9.202 ± 0.200°, 13.394 ± 0.200°, 14.212 ± 0.200°, 15.516 ± 0.200°, and 19.917 ± 0.200°;
(3) the crystal form C has an X-ray powder diffraction pattern comprising characteristic diffraction peaks at the following 2θ angles: 6.334 ± 0.200°, 8.399 ± 0.200°, 9.435 ± 0.200°, 12.060 ± 0.200°, and 18.126 ± 0.200°;
(4) the crystal form D has an X-ray powder diffraction pattern comprising characteristic diffraction peaks at the following 2θ angles: 9.136 ± 0.200°, 9.908 ± 0.200°, 17.787 ± 0.200°, 18.329 ± 0.200°, and 24.298 ± 0.200°;
(5) the crystal form E has an X-ray powder diffraction pattern comprising characteristic diffraction peaks at the following 2θ angles: 5.987 ± 0.200°, 9.928 ± 0.200°, 11.998 ± 0.200°, 13.499 ± 0.200°, and 18.044 ± 0.200°.

2. The crystal form A, B, C, D or E of the compound of formula (I) according to claim 1, wherein (1) the X-ray powder diffraction pattern of the crystal form A, expressed by a 2θ angle, comprises at least 6, 7, or 8 diffraction peaks selected from: 6.917 ± 0.200°, 10.655 ± 0.200°, 11.988 ± 0.200°, 14.481 ± 0.200°, 16.055 ± 0.200°, 17.653 ± 0.200°, 18.356 ± 0.200°, and 20.083 ± 0.200°;
(2) the X-ray powder diffraction pattern of the crystal form B, expressed by a 2θ angle, comprises at least 6, 7, or 8 diffraction peaks selected from: 9.202 ± 0.200°, 10.968 ± 0.200°, 13.394 ± 0.200°, 14.212 ± 0.200°, 15.516 ± 0.200°, 16.483 ± 0.200°, 18.306 ± 0.200°, and 19.917 ± 0.200°;
(3) the X-ray powder diffraction pattern of the crystal form C, expressed by a 2θ angle, comprises at least 6, 7, or 8 diffraction peaks selected from: 4.883 ± 0.200°, 6.334 ± 0.200°, 8.399 ± 0.200°, 8.928 ± 0.200°, 9.435 ± 0.200°, 11.168 ± 0.200°, 12.060 ± 0.200°, and 18.126 ± 0.200°.

3. The crystal form A, B, C, D or E of the compound of formula (I) according to claim 1, wherein (1) the X-ray powder diffraction pattern of the crystal form A comprises characteristic diffraction peaks at the following 2θ angles: 6.917 ± 0.200°, 10.655 ± 0.200°, 11.988 ± 0.200°, 14.481 ± 0.200°, 16.055 ± 0.200°, 17.653 ± 0.200°, 18.356 ± 0.200°, and 20.083 ± 0.200°;
(2) the X-ray powder diffraction pattern of the crystal form B has characteristic diffraction peaks at the following 2θ angles: 9.202 ± 0.200°, 10.968 ± 0.200°, 13.394 ± 0.200°, 14.212 ± 0.200°, 15.516 ± 0.200°, 16.483 ± 0.200°, 18.306 ± 0.200°, and 19.917 ± 0.200°;
(3) the X-ray powder diffraction pattern of the crystal form C comprises characteristic diffraction peaks at the following 2θ angles: 4.883 ± 0.200°, 6.334 ± 0.200°, 8.399 ± 0.200°, 8.928 ± 0.200°, 9.435 ± 0.200°, 11.168 ± 0.200°, 12.060 ± 0.200°, and 18.126 ± 0.200°;
(4) the X-ray powder diffraction pattern of the crystal form E comprises characteristic diffraction peaks at the following 2θ angles: 5.987 ± 0.200°, 9.928 ± 0.200°, 11.998 ± 0.200°, 13.499 ± 0.200°, 18.044 ± 0.200°, 19.492 ± 0.200°, and 25.173 ± 0.200°.

4. The crystal form A, B, C, D or E of the compound of formula (I) according to claim 1, wherein (1) the X-ray powder diffraction pattern of the crystal form A, expressed by a 2θ angle, comprises at least 12, 13, 14, 15, or 16 diffraction peaks selected from: 6.917 ± 0.200°, 10.655 ± 0.200°, 11.988 ± 0.200°, 13.837 ± 0.200°, 14.481 ± 0.200°, 16.055 ± 0.200°, 17.653 ± 0.200°, 18.356 ± 0.200°, 20.083 ± 0.200°, 20.839 ± 0.200°, 21.388 ± 0.200°, 22.379 ± 0.200°, 24.577 ± 0.200°, 25.104 ± 0.200°, 26.402 ± 0.200°, and 31.540 ± 0.200°;
(2) the X-ray powder diffraction pattern of the crystal form B, expressed by a 2θ angle, comprises at least 12, 13, 14, 15, or 16 diffraction peaks selected from: 5.459 ± 0.200°, 7.096 ± 0.200°, 9.202 ± 0.200°, 10.968 ± 0.200°, 13.394 ± 0.200°, 14.212 ± 0.200°, 15.516 ± 0.200°, 16.483 ± 0.200°, 18.306 ± 0.200°, 19.488 ± 0.200°, 19.917 ± 0.200°, 20.681 ± 0.200°, 21.526 ± 0.200°, 22.409 ± 0.200°, 24.622 ± 0.200°, and 25.661 ± 0.200°;
(3) the X-ray powder diffraction pattern of the crystal form C, expressed by a 2θ angle, comprises at least 12, 13, 14, 15, or 16 diffraction peaks selected from: 4.883 ± 0.200°, 6.334 ± 0.200°, 8.399 ± 0.200°, 8.928 ± 0.200°, 9.435 ± 0.200°, 11.168 ± 0.200°, 12.060 ± 0.200°, 12.988 ± 0.200°, 14.821 ± 0.200°, 16.182 ± 0.200°, 18.126 ± 0.200°, 18.545 ± 0.200°, 19.474 ± 0.200°, 20.199 ± 0.200°, 24.294 ± 0.200°, and 25.697 ± 0.200°.

5. The crystal form A, B, C, D or E of the compound of formula (I) according to claim 1, wherein (1) the X-ray powder diffraction pattern of the crystal form A comprises characteristic diffraction peaks at the following 2θ angles: 6.917 ± 0.200°, 10.655 ± 0.200°, 11.988 ± 0.200°, 13.837 ± 0.200°, 14.481 ± 0.200°, 16.055 ± 0.200°, 17.653 ± 0.200°, 18.356 ± 0.200°, 20.083 ± 0.200°, 20.839 ± 0.200°, 21.388 ± 0.200°, 22.379 ± 0.200°, 24.577 ± 0.200°, 25.104 ± 0.200°, 26.402 ± 0.200°, and 31.540 ± 0.200°;
(2) the X-ray powder diffraction pattern of the crystal form B comprises characteristic diffraction peaks at the following 2θ angles: 5.459 ± 0.200°, 7.096 ± 0.200°, 9.202 ± 0.200°, 10.968 ± 0.200°, 13.394 ± 0.200°, 14.212 ± 0.200°, 15.516 ± 0.200°, 16.483 ± 0.200°, 18.306 ± 0.200°, 19.488 ± 0.200°, 19.917 ± 0.200°, 20.681 ± 0.200°, 21.526 ± 0.200°, 22.409 ± 0.200°, 24.622 ± 0.200°, and 25.661 ± 0.200°;
(3) the X-ray powder diffraction pattern of the crystal form C comprises characteristic diffraction peaks at the following 2θ angles: 4.883 ± 0.200°, 6.334 ± 0.200°, 8.399 ± 0.200°, 8.928 ± 0.200°, 9.435 ± 0.200°, 11.168 ± 0.200°, 12.060 ± 0.200°, 12.988 ± 0.200°, 14.821 ± 0.200°, 16.182 ± 0.200°, 18.126 ± 0.200°, 18.545 ± 0.200°, 19.474 ± 0.200°, 20.199 ± 0.200°, 24.294 ± 0.200°, and 25.697 ± 0.200°.

6. The crystal form A, B, C, D or E of the compound of formula (I) according to claim 1, wherein (1) the crystal form A has a differential scanning calorimetry curve with an endothermic peak at 173.5°C ± 3°C;
(2) the crystal form B has a differential scanning calorimetry curve with an endothermic peak at 128.4°C ± 3°C;
(3) the crystal form C has a differential scanning calorimetry curve with an endothermic peak at 113.3°C ± 3°C;
(4) the crystal form D has a differential scanning calorimetry curve with an endothermic peak at 113.3°C ± 3°C;
(5) the crystal form E has a differential scanning calorimetry curve with an endothermic peak at 113.7°C ± 3°C.

7. The crystal form A, B, C, D or E of the compound of formula (I) according to claim 1, wherein (1) the crystal form A has a thermogravimetric analysis curve with a weight loss of 0.00% at 150.0°C ± 3°C;
(2) the crystal form B has a thermogravimetric analysis curve with a weight loss of 9.77% at 150.0°C ± 3°C;
(3) the crystal form C has a thermogravimetric analysis curve with a weight loss of 3.52% at 110.0°C ± 3°C and a weight loss of 10.58% at 150.0°C ± 3°C;
(4) the crystal form D has a thermogravimetric analysis curve with a weight loss of 6.59% at 90.0°C ± 3°C and a weight loss of 15.60% at 150.0°C ± 3°C;
(5) the crystal form E has a thermogravimetric analysis curve with a weight loss of 13.97% at 150.0°C ± 3°C.

8. The crystal form A, B, C, D or E of the compound of formula (I) according to claim 1, wherein (1) the crystal form B exist in the form of a solvate crystal;
(2) the crystal form C exist in the form of a solvate crystal;
(3) the crystal form D exist in the form of a solvate crystal;
(4) the crystal form E exist in the form of a solvate crystal.

9. A crystalline composition comprising the crystal form A, B, C, D or E according to any one of claims 1 to 8.

10. A pharmaceutical composition comprising the crystal form A, B, C, D or E according to any one of claims 1 to 8, or the crystalline composition according to claim 9.

11. The crystal form A, B, C, D or E according to any one of claims 1 to 8, the crystalline composition according to claim 9, or the pharmaceutical composition according to claim 10 for use in treating coronavirus infection.

12. The crystal form A, B, C, D or E, the crystalline composition, or the pharmaceutical composition for use in treating coronavirus infection according to claim 11, wherein the coronavirus infection is SARS-CoV-2 virus infection.

## Patentansprüche

1. Eine Kristallform A, B, C, D oder E einer Verbindung der Formel (I), 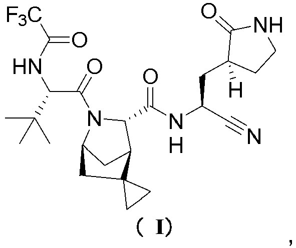
wobei (1) die Kristallform A ein Röntgen-Pulverbeugungsmuster aufweist, das charakteristische Beugungspeaks bei den folgenden 2-θ-Winkeln beinhaltet: 10,655 ± 0,200°, 11,988 ± 0,200°, 16,055 ± 0,200°, 18,356 ± 0,200° und 20,083 ± 0,200°;
(2) die Kristallform B ein Röntgen-Pulverbeugungsmuster aufweist, das charakteristische Beugungspeaks bei den folgenden 2-θ-Winkeln beinhaltet: 9,202 ± 0,200°, 13,394 ± 0,200°, 14,212 ± 0,200°, 15,516 ± 0,200° und 19,917 ± 0,200°;
(3) die Kristallform C ein Röntgen-Pulverbeugungsmuster aufweist, das charakteristische Beugungspeaks bei den folgenden 2-θ-Winkeln beinhaltet: 6,334 ± 0,200°, 8,399 ± 0,200°, 9,435 ± 0,200°, 12,060 ± 0,200° und 18,126 ± 0,200°;
(4) die Kristallform D ein Röntgen-Pulverbeugungsmuster aufweist, das charakteristische Beugungspeaks bei den folgenden 2-θ-Winkeln beinhaltet: 9,136 ± 0,200°, 9,908 ± 0,200°, 17,787 ± 0,200°, 18,329 ± 0,200° und 24,298 ± 0,200°;
(5) die Kristallform E ein Röntgen-Pulverbeugungsmuster aufweist, das charakteristische Beugungspeaks bei den folgenden 2-θ-Winkeln beinhaltet: 5,987 ± 0,200°, 9,928 ± 0,200°, 11,998 ± 0,200°, 13,499 ± 0,200° und 18,044 ± 0,200°.

2. Kristallform A, B, C, D oder E der Verbindung der Formel (I) gemäß Anspruch 1, wobei
(1) das Röntgen-Pulverbeugungsmuster der Kristallform A, ausgedrückt durch einen 2-θ-Winkel, mindestens 6, 7 oder 8 Beugungspeaks beinhaltet, die aus den folgenden ausgewählt sind: 6,917 ± 0,200°, 10,655 ± 0,200°, 11,998 ± 0,200°, 14,481 ± 0,200°, 16,055 ± 0,200°, 17,653 ± 0,200°, 18,356 ± 0,200° und 20,083 ± 0,200°;
(2) das Röntgen-Pulverbeugungsmuster der Kristallform B, ausgedrückt durch einen 2-θ-Winkel, mindestens 6, 7 oder 8 Beugungspeaks beinhaltet, die aus den folgenden ausgewählt sind: 9,202 ± 0,200°, 10,968 ± 0,200°, 13,394 ± 0,200°, 14,212 ± 0,200°, 15,516 ± 0,200°, 16,483 ± 0,200°, 18,306 ± 0,200° und 19,917 ± 0,200°;
(3) das Röntgen-Pulverbeugungsmuster der Kristallform C, ausgedrückt durch einen 2-θ-Winkel, mindestens 6, 7 oder 8 Beugungspeaks beinhaltet, die aus den folgenden ausgewählt sind: 4,883 ± 0,200°, 6,334 ± 0,200°, 8,399 ± 0,200°, 8,928 ± 0,200°, 9,435 ± 0,200°, 11,168 ± 0,200°, 12,060 ± 0,200° und 18,126 ± 0,200°.

3. Kristallform A, B, C, D oder E der Verbindung der Formel (I) gemäß Anspruch 1, wobei
(1) das Röntgen-Pulverbeugungsmuster der Kristallform A charakteristische Beugungspeaks bei den folgenden 2-θ-Winkeln beinhaltet: 6,917 ± 0,200°, 10,655 ± 0,200°, 11,998 ± 0,200°, 14,481 ± 0,200°, 16,055 ± 0,200°, 17,653 ± 0,200°, 18,356 ± 0,200° und 20,083 ± 0,200°;
(2) das Röntgen-Pulverbeugungsmuster der Kristallform B charakteristische Beugungspeaks bei den folgenden 2-θ-Winkeln aufweist: 9,202 ± 0,200°, 10,968 ± 0,200°, 13,394 ± 0,200°, 14,212 ± 0,200°, 15,516 ± 0,200°, 16,483 ± 0,200°, 18,306 ± 0,200° und 19,917 ± 0,200°;
(3) das Röntgen-Pulverbeugungsmuster der Kristallform C charakteristische Beugungspeaks bei den folgenden 2-θ-Winkeln beinhaltet: 4,883 ± 0,200°, 6,334 ± 0,200°, 8,399 ± 0,200°, 8,928 ± 0,200°, 9,435 ± 0,200°, 11,168 ± 0,200°, 12,060 ± 0,200° und 18,126 ± 0,200°;
(4) das Röntgen-Pulverbeugungsmuster der Kristallform E charakteristische Beugungspeaks bei den folgenden 2-θ-Winkeln beinhaltet: 5,987 ± 0,200°, 9,928 ± 0,200°, 11,998 ± 0,200°, 13,499 ± 0.200°, 18,044 ± 0,200°, 19,492 ± 0,200° und 25,173 ± 0,200°.

4. Kristallform A, B, C, D oder E der Verbindung der Formel (I) gemäß Anspruch 1, wobei
(1) das Röntgen-Pulverbeugungsmuster der Kristallform A, ausgedrückt durch einen 2-θ-Winkel, mindestens 12, 13, 14, 15 oder 16 Beugungspeaks beinhaltet, die aus den folgenden ausgewählt sind: 6,917 ± 0,200°, 10,655 ± 0,200°, 11,998 ± 0,200°, 13,837 ± 0.200°, 14,481 ± 0,200°, 16,055 ± 0,200°, 17,653 ± 0,200°, 18,356 ± 0.200°, 20,083 ± 0,200°, 20,839 ± 0,200°, 21,388 ± 0,200°, 22,379 ± 0,200°, 24,577 ± 0,200°, 25,104 ± 0,200°, 26,402 ± 0,200° und 31,540 ± 0,200°;
(2) das Röntgen-Pulverbeugungsmuster der Kristallform B, ausgedrückt durch einen 2-θ-Winkel, mindestens 12, 13, 14, 15 oder 16 Beugungspeaks beinhaltet, die aus den folgenden ausgewählt sind: 5,459 ± 0,200°, 7,096 ± 0,200°, 9,202 ± 0,200°, 10,968 ± 0.200°, 13,394 ± 0,200°, 14,212 ± 0,200°, 15,516 ± 0,200°, 16,483 ± 0.200°, 18,306 ± 0,200°, 19,488 ± 0,200°, 19,917 ± 0,200°, 20,681 ± 0,200°, 21,526 ± 0,200°, 22,409 ± 0,200°, 24,622 ± 0,200° und 25,661 ± 0,200°;
(3) das Röntgen-Pulverbeugungsmuster der Kristallform C, ausgedrückt durch einen 2-θ-Winkel, mindestens 12, 13, 14, 15 oder 16 Beugungspeaks beinhaltet, die aus den folgenden ausgewählt sind: 4,883 ± 0,200°, 6,334 ± 0,200°, 8,399 ± 0,200°, 8,928 ± 0.200°, 9,435 ± 0,200°, 11,168 ± 0,200°, 12,060 ± 0,200°, 12,988 ± 0.200°, 14,821 ± 0,200°, 16,182 ± 0,200°, 18,126 ± 0,200°, 18,545 ± 0,200°, 19,474 ± 0,200°, 20,199 ± 0,200°, 24,294 ± 0,200° und 25,697 ± 0,200°.

5. Kristallform A, B, C, D oder E der Verbindung der Formel (I) gemäß Anspruch 1, wobei
(1) das Röntgen-Pulverbeugungsmuster der Kristallform A charakteristische Beugungspeaks bei den folgenden 2-θ-Winkeln beinhaltet: 6,917 ± 0,200°, 10,655 ± 0,200°, 11,998 ± 0,200°, 13,837 ± 0.200°, 14,481 ± 0,200°, 16,055 ± 0,200°, 17,653 ± 0,200°, 18,356 ± 0.200°, 20,083 ± 0,200°, 20,839 ± 0,200°, 21,388 ± 0,200°, 22,379 ± 0,200°, 24,577 ± 0,200°, 25,104 ± 0,200°, 26,402 ± 0,200° und 31,540 ± 0,200°;
(2) das Röntgen-Pulverbeugungsmuster der Kristallform B charakteristische Beugungspeaks bei den folgenden 2-θ-Winkeln beinhaltet: 5,459 ± 0,200°, 7,096 ± 0,200°, 9,202 ± 0,200°, 10,968 ± 0.200°, 13,394 ± 0,200°, 14,212 ± 0,200°, 15,516 ± 0,200°, 16,483 ± 0.200°, 18,306 ± 0,200°, 19,488 ± 0,200°, 19,917 ± 0,200°, 20,681 ± 0,200°, 21,526 ± 0,200°, 22,409 ± 0,200°, 24,622 ± 0,200° und 25,661 ± 0,200°;
(3) das Röntgen-Pulverbeugungsmuster der Kristallform C charakteristische Beugungspeaks bei den folgenden 2-θ-Winkeln beinhaltet: 4,883 ± 0,200°, 6,334 ± 0,200°, 8,399 ± 0,200°, 8,928 ± 0.200°, 9,435 ± 0,200°, 11,168 ± 0,200°, 12,060 ± 0,200°, 12,988 ± 0.200°, 14,821 ± 0,200°, 16,182 ± 0,200°, 18,126 ± 0,200°, 18,545 ± 0,200°, 19,474 ± 0,200°, 20,199 ± 0,200°, 24,294 ± 0,200° und 25,697 ± 0,200°.

6. Kristallform A, B, C, D oder E der Verbindung der Formel (I) gemäß Anspruch 1, wobei
(1) die Kristallform A bei dynamischer Differenzkalorimetrie eine Kurve mit einem endothermen Peak bei 173,5 °C ± 3 °C aufweist;
(2) die Kristallform B bei dynamischer Differenzkalorimetrie eine Kurve mit einem endothermen Peak bei 128,4 °C ± 3 °C aufweist;
(3) die Kristallform C bei dynamischer Differenzkalorimetrie eine Kurve mit einem endothermen Peak bei 113,3 °C ± 3 °C aufweist;
(4) die Kristallform D bei dynamischer Differenzkalorimetrie eine Kurve mit einem endothermen Peak bei 113,3 °C ± 3 °C aufweist;
(5) die Kristallform E bei dynamischer Differenzkalorimetrie eine Kurve mit einem endothermen Peak bei 113,7 °C ± 3 °C aufweist.

7. Kristallform A, B, C, D oder E der Verbindung der Formel (I) gemäß Anspruch 1, wobei
(1) die Kristallform A bei thermogravimetrischer Analyse eine Kurve mit einem Gewichtsverlust von 0,00 % bei 150,0 °C ± 3 °C aufweist;
(2) die Kristallform B bei thermogravimetrischer Analyse eine Kurve mit einem Gewichtsverlust von 9,77 % bei 150,0 °C ± 3 °C aufweist;
(3) die Kristallform C bei thermogravimetrischer Analyse eine Kurve mit einem Gewichtsverlust von 3,52 % bei 110,0 °C ± 3 °C und einem Gewichtsverlust von 10,58 % bei 150,3 °C ± 3 °C aufweist;
(4) die Kristallform D bei thermogravimetrischer Analyse eine Kurve mit einem Gewichtsverlust von 6,59 % bei 90,0 °C ± 3 °C und einem Gewichtsverlust von 15,60 % bei 150,0 °C ± 3 °C aufweist;
(5) die Kristallform E bei thermogravimetrischer Analyse eine Kurve mit einem Gewichtsverlust von 13,97 % bei 150,0 °C ± 3 °C aufweist.

8. Kristallform A, B, C, D oder E der Verbindung der Formel (I) gemäß Anspruch 1, wobei
(1) die Kristallform B in Form eines Solvatkristalls besteht;
(2) die Kristallform C in Form eines Solvatkristalls besteht;
(3) die Kristallform D in Form eines Solvatkristalls besteht;
(4) die Kristallform E in Form eines Solvatkristalls besteht.

9. Eine kristalline Zusammensetzung, die die Kristallform A, B, C, D oder E gemäß einem der Ansprüche 1 bis 8 beinhaltet.

10. Eine pharmazeutische Zusammensetzung, die die Kristallform A, B, C, D oder E gemäß einem der Ansprüche 1 bis 8 oder die kristalline Zusammensetzung gemäß Anspruch 9 beinhaltet.

11. Kristallform A, B, C, D oder E gemäß einem der Ansprüche 1 bis 8, kristalline Zusammensetzung gemäß Anspruch 9 oder pharmazeutische Zusammensetzung gemäß Anspruch 10 zur Verwendung beim Behandeln einer Coronavirus-Infektion.

12. Kristallform A, B, C, D oder E, kristalline Zusammensetzung oder pharmazeutische Zusammensetzung zur Verwendung beim Behandeln einer Coronavirus-Infektion gemäß Anspruch 11, wobei die Coronavirus-Infektion eine SARS-CoV-2-Virusinfektion ist.

## Revendications

1. Une forme cristalline A, B, C, D ou E d'un composé de formule (I), 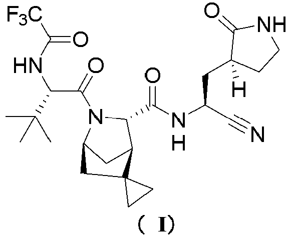
(1) la forme cristalline A ayant un diagramme de diffraction de rayons X sur poudres comprenant des pics de diffraction caractéristiques aux angles 2θ suivants : 10,655 ± 0,200°, 11,988 ± 0,200°, 16,055 ± 0,200°, 18,356 ± 0,200°, et 20,083 ± 0,200° ;
(2) la forme cristalline B ayant un diagramme de diffraction de rayons X sur poudres comprenant des pics de diffraction caractéristiques aux angles 2θ suivants : 9,202 ± 0,200°, 13,394 ± 0,200°, 14,212 ± 0,200°, 15,516 ± 0,200°, et 19,917 ± 0,200° ;
(3) la forme cristalline C ayant un diagramme de diffraction de rayons X sur poudres comprenant des pics de diffraction caractéristiques aux angles 2θ suivants : 6,334 ± 0,200°, 8,399 ± 0,200°, 9,435 ± 0,200°, 12,060 ± 0,200°, et 18,126 ± 0,200° ;
(4) la forme cristalline D ayant un diagramme de diffraction de rayons X sur poudres comprenant des pics de diffraction caractéristiques aux angles 2θ suivants : 9,136 ± 0,200°, 9,908 ± 0,200°, 17,787 ± 0,200°, 18,329 ± 0,200°, et 24,298 ± 0,200° ;
(5) la forme cristalline E ayant un diagramme de diffraction de rayons X sur poudres comprenant des pics de diffraction caractéristiques aux angles 2θ suivants : 5,987 ± 0,200°, 9,928 ± 0,200°, 11,998 ± 0,200°, 13,499 ± 0,200°, et 18,044 ± 0,200°.

2. La forme cristalline A, B, C, D ou E du composé de formule (I) selon la revendication 1, dans laquelle (1) le diagramme de diffraction de rayons X sur poudres de la forme cristalline A, exprimé par un angle 2θ, comprend au moins 6, 7, ou 8 pics de diffraction sélectionnés parmi : 6,917 ± 0,200°, 10,655 ± 0,200°, 11,988 ± 0,200°, 14,481 ± 0,200°, 16,055 ± 0,200°, 17,653 ± 0,200°, 18,356 ± 0,200°, et 20,083 ± 0,200° ;
(2) le diagramme de diffraction de rayons X sur poudres de la forme cristalline B, exprimé par un angle 2θ, comprend au moins 6, 7, ou 8 pics de diffraction sélectionnés parmi : 9,202 ± 0,200°, 10,968 ± 0,200°, 13,394 ± 0,200°, 14,212 ± 0,200°, 15,516 ± 0,200°, 16,483 ± 0,200°, 18,306 ± 0,200°, et 19,917 ± 0,200° ;
(3) le diagramme de diffraction de rayons X sur poudres de la forme cristalline C, exprimé par un angle 2θ, comprend au moins 6, 7, ou 8 pics de diffraction sélectionnés parmi : 4,883 ± 0,200°, 6,334 ± 0,200°, 8,399 ± 0,200°, 8,928 ± 0,200°, 9,435 ± 0,200°, 11,168 ± 0,200°, 12,060 ± 0,200°, et 18,126 ± 0,200°.

3. La forme cristalline A, B, C, D ou E du composé de formule (I) selon la revendication 1, dans laquelle (1) le diagramme de diffraction de rayons X sur poudres de la forme cristalline A comprend des pics de diffraction caractéristiques aux angles 2θ suivants : 6,917 ± 0,200°, 10,655 ± 0,200°, 11,988 ± 0,200°, 14,481 ± 0,200°, 16,055 ± 0,200°, 17,653 ± 0,200°, 18,356 ± 0,200°, et 20,083 ± 0,200° ;
(2) le diagramme de diffraction de rayons X sur poudres de la forme cristalline B comprend des pics de diffraction caractéristiques aux angles 2θ suivants : 9,202 ± 0,200°, 10,968 ± 0,200°, 13,394 ± 0,200°, 14,212 ± 0,200°, 15,516 ± 0,200°, 16,483 ± 0,200°, 18,306 ± 0,200°, et 19,917 ± 0,200° ;
(3) le diagramme de diffraction de rayons X sur poudres de la forme cristalline C comprend des pics de diffraction caractéristiques aux angles 2θ suivants : 4,883 ± 0,200°, 6,334 ± 0,200°, 8,399 ± 0,200°, 8,928 ± 0,200°, 9,435 ± 0,200°, 11,168 ± 0,200°, 12,060 ± 0,200°, et 18,126 ± 0,200° ;
(4) le diagramme de diffraction de rayons X sur poudres de la forme cristalline E comprend des pics de diffraction caractéristiques aux angles 2θ suivants : 5,987 ± 0,200°, 9,928 ± 0,200°, 11,998 ± 0,200°, 13,499 ± 0,200°, 18,044 ± 0,200°, 19,492 ± 0,200°, et 25,173 ± 0,200°.

4. La forme cristalline A, B, C, D ou E du composé de formule (I) selon la revendication 1, dans laquelle (1) le diagramme de diffraction de rayons X sur poudres de la forme cristalline A, exprimé par un angle 2θ, comprend au moins 12, 13, 14, 15, ou 16 pics de diffraction sélectionnés parmi : 6,917 ± 0,200°, 10,655 ± 0,200°, 11,988 ± 0,200°, 13,837 ± 0,200°, 14,481 ± 0,200°, 16,055 ± 0,200°, 17,653 ± 0,200°, 18,356 ± 0,200°, 20,083 ± 0,200°, 20,839 ± 0,200°, 21,388 ± 0,200°, 22,379 ± 0,200°, 24,577 ± 0,200°, 25,104 ± 0,200°, 26,402 ± 0,200°, et 31,540 ± 0,200° ;
(2) le diagramme de diffraction de rayons X sur poudres de la forme cristalline B, exprimé par un angle 2θ, comprend au moins 12, 13, 14, 15, ou 16 pics de diffraction sélectionnés parmi : 5,459 ± 0,200°, 7,096 ± 0,200°, 9,202 ± 0,200°, 10,968 ± 0,200°, 13,394 ± 0,200°, 14,212 ± 0,200°, 15,516 ± 0,200°, 16,483 ± 0,200°, 18,306 ± 0,200°, 19,488 ± 0,200°, 19,917 ± 0,200°, 20,681 ± 0,200°, 21,526 ± 0,200°, 22,409 ± 0,200°, 24,622 ± 0,200°, et 25,661 ± 0,200° ;
(3) le diagramme de diffraction de rayons X sur poudres de la forme cristalline C, exprimé par un angle 2θ, comprend au moins 12, 13, 14, 15, ou 16 pics de diffraction sélectionnés parmi : 4,883 ± 0,200°, 6,334 ± 0,200°, 8,399 ± 0,200°, 8,928 ± 0,200°, 9,435 ± 0,200°, 11,168 ± 0,200°, 12,060 ± 0,200°, 12,988 ± 0,200°, 14,821 ± 0,200°, 16,182 ± 0,200°, 18,126 ± 0,200°, 18,545 ± 0,200°, 19,474 ± 0,200°, 20,199 ± 0,200°, 24,294 ± 0,200°, et 25,697 ± 0,200°.

5. La forme cristalline A, B, C, D ou E du composé de formule (I) selon la revendication 1, dans laquelle (1) le diagramme de diffraction de rayons X sur poudres de la forme cristalline A comprend des pics de diffraction caractéristiques aux angles 2θ suivants : 6,917 ± 0,200°, 10,655 ± 0,200°, 11,988 ± 0,200°, 13,837 ± 0,200°, 14,481 ± 0,200°, 16,055 ± 0,200°, 17,653 ± 0,200°, 18,356 ± 0,200°, 20,083 ± 0,200°, 20,839 ± 0,200°, 21,388 ± 0,200°, 22,379 ± 0,200°, 24,577 ± 0,200°, 25,104 ± 0,200°, 26,402 ± 0,200°, et 31,540 ± 0,200° ;
(2) le diagramme de diffraction de rayons X sur poudres de la forme cristalline B comprend des pics de diffraction caractéristiques aux angles 2θ suivants : 5,459 ± 0,200°, 7,096 ± 0,200°, 9,202 ± 0,200°, 10,968 ± 0,200°, 13,394 ± 0,200°, 14,212 ± 0,200°, 15,516 ± 0,200°, 16,483 ± 0,200°, 18,306 ± 0,200°, 19,488 ± 0,200°, 19,917 ± 0,200°, 20,681 ± 0,200°, 21,526 ± 0,200°, 22,409 ± 0,200°, 24,622 ± 0,200°, et 25,661 ± 0,200° ;
(3) le diagramme de diffraction de rayons X sur poudres de la forme cristalline C comprend des pics de diffraction caractéristiques aux angles 2θ suivants : 4,883 ± 0,200°, 6,334 ± 0,200°, 8,399 ± 0,200°, 8,928 ± 0,200°, 9,435 ± 0,200°, 11,168 ± 0,200°, 12,060 ± 0,200°, 12,988 ± 0,200°, 14,821 ± 0,200°, 16,182 ± 0,200°, 18,126 ± 0,200°, 18,545 ± 0,200°, 19,474 ± 0,200°, 20,199 ± 0,200°, 24,294 ± 0,200°, et 25,697 ± 0,200°.

6. La forme cristalline A, B, C, D ou E du composé de formule (I) selon la revendication 1,
(1) la forme cristalline A ayant une courbe de calorimétrie différentielle à balayage avec un pic endothermique à 173,5 °C ± 3 °C ;
(2) la forme cristalline B ayant une courbe de calorimétrie différentielle à balayage avec un pic endothermique à 128,4 °C ± 3 °C ;
(3) la forme cristalline C ayant une courbe de calorimétrie différentielle à balayage avec un pic endothermique à 113,3 °C ± 3 °C ;
(4) la forme cristalline D ayant une courbe de calorimétrie différentielle à balayage avec un pic endothermique à 113,3 °C ± 3 °C ;
(5) la forme cristalline E ayant une courbe de calorimétrie différentielle à balayage avec un pic endothermique à 113,7 °C ± 3 °C.

7. La forme cristalline A, B, C, D ou E du composé de formule (I) selon la revendication 1,
(1) la forme cristalline A ayant une courbe d'analyse thermogravimétrique avec une perte de poids de 0,00 % à 150,0 °C ± 3 °C ;
(2) la forme cristalline B ayant une courbe d'analyse thermogravimétrique avec une perte de poids de 9,77 % à 150,0 °C ± 3 °C ;
(3) la forme cristalline C ayant une courbe d'analyse thermogravimétrique avec une perte de poids de 3,52 % à 110,0 °C ± 3 °C et une perte de poids de 10,58 % à 150,0 °C ± 3 °C ;
(4) la forme cristalline D ayant une courbe d'analyse thermogravimétrique avec une perte de poids de 6,59 % à 90,0 °C ± 3 °C et une perte de poids de 15,60 % à 150,0 °C ± 3 °C ;
(5) la forme cristalline E ayant une courbe d'analyse thermogravimétrique avec une perte de poids de 13,97 % à 150,0 °C ± 3 °C.

8. La forme cristalline A, B, C, D ou E du composé de formule (I) selon la revendication 1,
(1) la forme cristalline B existant sous la forme d'un cristal de solvate ;
(2) la forme cristalline C existant sous la forme d'un cristal de solvate ;
(3) la forme cristalline D existant sous la forme d'un cristal de solvate ;
(4) la forme cristalline E existant sous la forme d'un cristal de solvate.

9. Une composition cristalline comprenant la forme cristalline A, B, C, D ou E selon l'une quelconque des revendications 1 à 8.

10. Une composition pharmaceutique comprenant la forme cristalline A, B, C, D ou E selon l'une quelconque des revendications 1 à 8, ou la composition cristalline selon la revendication 9.

11. La forme cristalline A, B, C, D ou E selon l'une quelconque des revendications 1 à 8, la composition cristalline selon la revendication 9, ou la composition pharmaceutique selon la revendication 10 destinée à une utilisation dans le traitement d'une infection à coronavirus.

12. La forme cristalline A, B, C, D ou E, la composition cristalline, ou la composition pharmaceutique destinée à une utilisation dans le traitement d'une infection à coronavirus selon la revendication 11, dans laquelle l'infection à coronavirus est une infection au virus SARS-CoV-2.
